# EUROPEAN PATENT SPECIFICATION

(11) **EP 1 773 740 B1**
(45) Date of publication and mention of the grant of the patent: **18.02.2009**
(21) Application number: 05754305.0
(22) Date of filing: 24.06.2005
(51) Int. Cl.: A61K 31/662, A61P 25/00, A61P 25/28, C07F 9/30

(54) **NEUROLOGICALLY-ACTIVE COMPOUNDS**
NEUROLOGISCH AKTIVE VERBINDUNGEN
COMPOSES PRESENTANT UNE ACTIVITE NEUROLOGIQUE

(30) Priority: 25.06.2004 AU 2004903472
(43) Date of publication of application: 18.04.2007
(73) Proprietor: THE UNIVERSITY OF SYDNEY, Sydney, New South Wales 2006 (AU); Polychip Pharmaceuticals Pty. Ltd., Toorak, VIC 3142 (AU)
(72) Inventor: CHEBIB, Mary, Sydney, NSW 2040 (AU); KUMAR, Rohan John, Sydney, NSW 2036 (AU); JOHNSTON, Graham, Sydney, NSW 2069 (AU); HANRAHAN, Jane, Sydney, NSW 2042 (AU)
(74) Representative: MacLean, Martin Robert
(86) International application number: PCT/AU2005/000928
(87) International publication number: WO 2006/000043

(56) References cited:
- WO-A1-03/045897
- CRITTENDEN, DEBORAH L. ET AL: "Stabilization of Zwitterions in Solution: Phosphinic and Phosphonic Acid GABA Analogues" JOURNAL OF PHYSICAL CHEMISTRY A , 109(37), 8398-8409 CODEN: JPCAFH; ISSN: 1089-5639, 2005, XP002445627

## Description

### Technical Field

This invention relates to neurologically-active compounds. In particular, the invention relates to compounds that are antagonists of GABA_{C} receptors, pharmaceutical compositions comprising the compounds and a method of enhancing the cognitive activity of an animal using the compounds.

### Background of the Invention

γ-Aminobutyric acid (GABA) is the main inhibitory neurotransmitter in the central nervous system and activates the three major sub-types of GABA receptors in the central nervous system, the GABA_{A}, GABA_{B} and GABA_{C} receptors. The pharmacology of GABA_{A} and GABA_{B} receptors has been extensively investigated. GABA_{C} receptors are the most recently described sub-type of GABA receptors, and are therefore the least studied sub-type.

GABA_{A} receptors are ligand gated Cl⁻ ion channels which are inhibited by the alkaloid bicuculline (Johnston, 1996a). GABA_{A} receptors are heterooligomeric made up of α, β, γ, δ and θ subunits. GABA_{B} receptors are transmembrane receptors coupled to second membrane messenger systems and Ca⁺ and K⁺ channels via G-proteins. These receptors are not blocked by bicuculline, but are activated by (-)-baclophen and 3-aminopropylphosphinic acid (CGP27492) and blocked by saclofen (Kerr and Ong, 1995).

GABA_{C} receptors (sometimes called GABA_{NANB} or ρ receptors) were first proposed when a series of conformationally restricted GABA analogues, including cis-4-aminocrotonic acid (CACA), that had bicuculline insensitive depression actions on neuronal activity, showed no affinity for [³H]baclofen binding sites in rat cerebellar membranes (Drew *et al*, 1984). GABA_{C} receptors with similar pharmacology were first found in neurons in rat retina (Feigenspan *et al,* 1993) and white perch retina (Qian *et al*, 1993).

Three major sub-types of GABA_{C} receptors are now known, namely ρ1, ρ2 and ρ3 (Chebib and Johnston, 2000).

GABA is a flexible compound, due to its rotation about the C2-C3 and C3-C4 bonds. It can exist in a range of low energy conformations (Johnston *et al,* 1978). Analogues of GABA in which two of these conformations have been restricted by the introduction of unsaturation in the form of a double bond at the C2-C3 position have been prepared. Two compounds that represent these restricted conformations are CACA and *trans*-4-aminocrotonic acid (TACA) (Johnston *et al,* 1975). CACA and TACA have fewer degrees of rotational freedom than GABA, and can only rotate about the C3-C4 bond (Johnston *et al,* 1978). CACA is a partially folded analogue of GABA. It has moderate activity at GABA_{C} receptors expressed in *Xenopus* oocytes, and although its agonist activity is weak, it is to date the most selective agonist at these receptors, having minimal activity on GABA_{A} and GABA_{B} receptors (Johnston, 1996b). TACA is an extended analogue of GABA. It has potent agonist activity at GABA_{C} receptors expressed in *Xenopus* oocytes; however, it is not selective, as it is also a potent GABA_{A} receptor agonist (Johnston, 1996b).

Woodward *et al* (1993) tested many GABA analogues on poly (A)⁺ RNA from mammalian retina expressed in *Xenopus* oocytes to determine a pharmaceutical profile for GABA_{C} receptors. From this study, it was found that phosphinic and methylphosphinic acid analogues of GABA, which are known to be potent GABA_{B} receptor agonists, were potent antagonists at GABA_{C} receptors. Several straight chain phosphinic, methylphosphinic and phosphonic acid analogues of GABA were shown to be potent antagonists at GABA_{C} receptors, including (3-aminopropyl)methylphosphinic acid (CGP35024, K_{B}=0.8 µM), 3-aminoporpylphosphinic acid (CGP27492, K_{B}=0.8 µM) and 3-aminopropylphosphonic acid (3-APA, K_{B}=1.8 µM) (Woodward *et al,* 1993). These agents are not selective for GABA_{C} receptors, as CGP35024 and CGP27492 are also very potent GABA_{B} receptor agonists, while 3-APA is a GABA_{B} receptor agonist.

One of the most potent and selective GABA_{C} receptor antagonists known is (1,2,5,6-tetrahydropyridine-4-yl)methylphosphinic acid (TPMPA, K_{B}=2.1µM) (Murata *et al,* 1996; Ragozzinno *et al,* 1996). TPMPA produces 50% inhibition of GABA_{C} receptor activation at 2.1 µM.

Chebib *et al,* 1997 demonstrated that TPMPA, the phosphinic and methyl phosphinic acid analogues of CACA and the closely related analogue TACA, and 3-aminopropyl-*n-*butyl-phosphinic acid (CGP36742), an orally active GABA_{B} receptor antagonist, are GABA_{C} receptor antagonists.

It has been shown that GABA_{C} receptor antagonists have therapeutic application.

Froestl *et al.,* 1995 investigated a series of GABA_{B} receptor antagonists in various memory and learning tests in rats and mice. Only one compound in this series, 3-aminopropyl-n-butyl-phosphinic acid (CGP36742), reversed age related deficits of old rats. The cognition-enhancing effects of this compound were confirmed in learning experiments in monkeys (Froestl *et al.,* 1995).

The cognition-enhancing effects of CGP36742 is not satisfactorily explained by its GABA_{B} antagonist properties, since much more potent GABA_{B} antagonists have been described that do not have these effects. Some of the present inventors have previously shown that CGP36742 has similar potency as a GABA_{C} antagonist to its potency as a GABA_{B} antagonist (50% inhibition of receptor activation being found at 38 µM and 62 µM against GABA_{B} and GABA_{C} receptors respectively) (Chebib *et al.,* 1997). None of the other potent GABA_{B} antagonists used in Froestl *et al.,* 1995 showed activity against GABA_{C} receptors. These findings indicate a role for GABA_{C} receptor antagonism in the cognition-enhancing properties of CGP36742 reported in Froestl *et al.,* 1995.

WO 98/58939 describes a method of enhancing the cognitive activity of an animal in need of such treatment, comprising the step of administering an effective amount of a compound which has GABA_{C} receptor antagonist activity to said animal. That document also describes a method of stimulating memory capacity, comprising the step of administering an effective amount of a compound which has GABA_{C} receptor antagonist activity to an animal in need of such treatment. The document also describes novel compounds having GABA_{C} receptor antagonist activity of the general formula I and general formula II as defined in that document.

Most of the GABA_{C} receptor antagonists described in the prior art, such as CGP36742, are non-selective, that is, they also have significant activity at the GABA_{A} or GABA_{B} receptors. The use of such compounds as GABA_{C} receptor antagonists may have undesirable side-effects via their action at the GABA_{A} and/or GABA_{B} receptors.

WO 03/045897 describes compounds of formula I and formula II as defined in that document. The compounds of formula I and formula II as defined in that document are selective GABA_{C} receptor antagonists.

It would be desirable to identify other selective antagonists of the GABA_{C} receptors. Selective antagonists of the GABA_{C} receptors are needed to provide therapeutic agents with a lower risk of unwanted side-effects due to actions at the GABA_{A} and/or GABA_{B} receptors than the prior art non-selective GABA_{C} receptor antagonists.

### Summary of the Invention

The inventors have found a class of compounds which are surprisingly selective GABA_{C} receptor antagonists.

In a first aspect, the present invention provides a compound of the formula I: 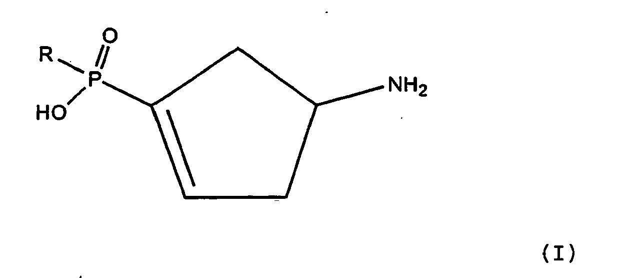 wherein R is methyl, ethyl, propyl, isopropyl, butyl, pentyl, neo-pentyl or cyclohexyl, or a salt or solvate thereof.

The compounds of formula I can exist in two stereoisomers. The present invention encompasses racemic mixtures of the stereoisomers of the compounds of formula I, as well as the individual stereoisomers.

The two stereoisomers of the compounds of the formula I are as follows:

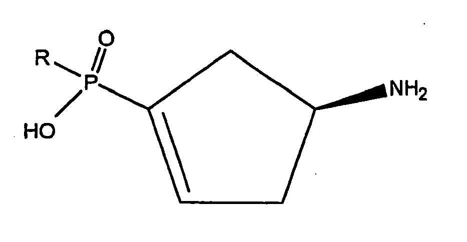

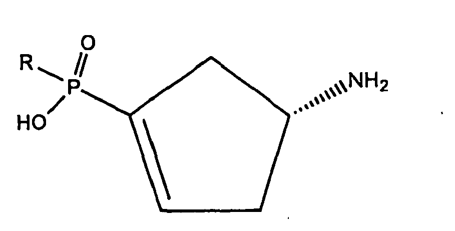

Each of these isomers is encompassed by the present invention.

The compounds of formula I are GABA analogues that are conformationally restricted by incorporating the backbone of GABA into a cyclopentene ring. This conformational restriction makes it difficult for the compounds to act at either GABA_{A} or GABA_{B} receptors, making these compounds selective GABA_{C} antagonists.

In a second aspect, the present invention provides in vitro method of selectively antagonising GABA_{C} receptors compared with GABA_{B} or GABA_{A} receptors, comprising the step of exposing the receptors to an effective amount of a compound of formula I as defined above or a pharmaceutically acceptable salt or solvate thereof.

The compounds of the present invention are suitable for the treatment of a variety of cognitive deficit conditions, dementias and memory impairment conditions, including but not limited to those associated with Alzheimer's disease, AIDS and schizophrenia.

While the invention is not restricted to the treatment of any particular animal species, in general, the animal is a human.

In another aspect, the present invention provides the use of a compound of formula I as defined above or a pharmaceutically acceptable salt or solvate thereof for the manufacture of a medicament for selectively antagonising GABA_{C} receptors compared with GABA_{B} or GABA_{A} receptors.

In another aspect, the present invention provides the use of a compound of formula I as defined above or a pharmaceutically acceptable salt or solvate thereof for the manufacture of a medicament for enhancing the cognitive activity of an animal.

In another aspect, the present invention provides the use of a compound of formula I as defined above or a pharmaceutically acceptable salt or solvate thereof for the manufacture of a medicament for stimulating memory capacity in an animal.

In another aspect, the present invention provides a pharmaceutical composition comprising a compound of formula I as defined above or a pharmaceutically acceptable salt or solvate thereof, and a pharmaceutically acceptable carrier.

### Detailed Description of Preferred Embodiments

The compounds of formula I are potent and selective antagonists of the GABA_{C} receptors, that is, they are potent antagonists of the GABA_{C} receptors and are more active at one or more of the GABA_{C} receptor sub-types than at the GABA_{A} and GABA_{B} receptors.

In some embodiments of the present invention, R is selected from the group consisting of methyl, ethyl, propyl, isopropyl and butyl. In one embodiment of the present invention, R is methyl. In another embodiment, R is ethyl. In other embodiments, R is propyl or isopropyl.

In a preferred embodiment of the present invention, R is butyl. The compound of formula I where R is butyl, has a butyl substituted phosphinic acid group in the 1-position of the cyclopentene ring corresponding to the carboxylic acid functionality of GABA. This substituent enhances the lipid solubility of the compound and thus increases the ability of the compound to cross the blood brain barrier.

As will be apparent to a person skilled in the art, various salts of the compounds of formula I can be prepared. Such salts include, for example, metal salts, ammonium salts, salts with an organic base, salts with an inorganic acid, and salts with an organic acid. Examples of metal salts include alkali metal salts, such as a sodium salt or a potassium salt. Examples of salts with an inorganic acid include salts with acids such as hydrochloric acid or hydrobromic acid. Examples of salts with organic acids include salts with acetic acid, trifluoroacetic acid, citric acid or formic acid.

The salts of the compounds of formula I are preferably pharmaceutically acceptable, but it will be appreciated that non-pharmaceutically acceptable salts also fall within the scope of the first aspect of the present invention. Non-pharmaceutically acceptable salts are useful as intermediates in the preparation of pharmaceutically acceptable salts. Examples of pharmaceutically acceptable salts include salts of pharmaceutically acceptable cations such as sodium, potassium, lithium, calcium, magnesium, ammonium and alkylammonium; acid addition salts of pharmaceutically acceptable inorganic acids such as hydrochloric, orthophosphoric, sulphuric, phosphoric, nitric, carbonic, boric, sulfamic and hydrobromic acids; or salts of pharmaceutically acceptable organic acids such as acetic, propionic, butyric, tartaric, maleic, hydroxymaleic, fumaric, citric, lactic, mucic, gluconic, benzoic, succinic, oxalic, phenylacetic, methanesulphonic, trihalomethanesulphonic, toluenesulphonic, benzenesulphonic, salicylic, sulphanilic, aspartic, glutamic, edetic, stearic, palmitic, oleic, lauric, pantothenic, tannic, ascorbic and valeric acids.

In addition, some of the compounds of the present invention may form solvates with water or common organic solvents. Such solvates are encompassed within the scope of the present invention.

The compounds of formula I as defined above or a pharmaceutically acceptable salt or solvate thereof, may be administered to an animal at any suitable dose and by any suitable route. The compounds may, for example, be administered orally or parenterally in dosage unit formulations containing conventional pharmaceutically acceptable carriers. The term "parenteral" as used herein includes subcutaneous injections, aerosol, intravenous, intramuscular, intrathecal, intracranial injection or infusion techniques. Oral administration is preferred because of its greater convenience and acceptability.

The pharmaceutical compositions of the present invention comprise at least one compound of formula I or a pharmaceutically acceptable salt or solvate thereof, together with one or more pharmaceutically acceptable carriers and optionally other therapeutic agents. Each carrier is "pharmaceutically acceptable" in the sense of being compatible with the other ingredients of the composition and not causing any substantially adverse reaction in the animal to which the composition is administered. The compositions may conveniently be presented in unit dosage form and may be prepared by methods well known in the art of pharmacy.

Suitable pharmaceutical compositions for administration by any desired route may be prepared by standard methods using pharmaceutically acceptable carriers known in the art, for example by reference to well known texts such as Remington: The Science and Practice of Pharmacy, 2000 (20th Edition), AR Gennaro (Ed), Lippincott Williams & Wilkins, Philadelphia, PA 19106-3621, USA or Australian Prescription Products Guide, 2000 (29th Edition), J Thomas (Ed), Australian Pharmaceutical Publishing Company Limited, Victoria, Australia.

Such methods include the step of bringing into association the active ingredient with the carrier which constitutes one or more accessory ingredients. In general, the compositions are prepared by uniformly and intimately bringing into association the active ingredient with liquid carriers or finely divided solid carriers or both, and then if necessary shaping the product.

The pharmaceutical compositions of the present invention may be formulated for oral administration as tablets, aqueous or oily suspensions, lozenges, troches, powders, granules, emulsions, capsules, syrups or elixirs. Compositions formulated for oral administration may contain one or more agents selected from the group of sweetening agents, flavouring agents, colouring agents and preserving agents in order to produce a pharmaceutically elegant and palatable preparation.

When the pharmaceutical composition of the present invention is in the form of a tablet suitable for oral administration, the tablet contains the compound of formula I as defined above or a pharmaceutically acceptable salt or solvate thereof in admixture with one or more pharmaceutically acceptable carriers. These pharmaceutically acceptable carriers may include, for example, (1) inert diluents, such as calcium carbonate, lactose, calcium phosphate or sodium phosphate; (2) granulating and disintegrating agents, such as com starch or alginic acid; (3) binding agents, such as starch, gelatine or acacia; and (4) lubricating agents, such as magnesium stearate, stearic acid or talc. The tablets may be uncoated or coated by known techniques to delay disintegration and absorption in the gastrointestinal tract and thereby provide a sustained action over a longer period. For example, a time delay material such as glyceryl monostearate or glycol distearate may be employed.

Pharmaceutical compositions for parenteral administration are typically in the form of sterile aqueous or non-aqueous solutions, suspensions, or emulsions. Suitable pharmaceutically acceptable carriers for such compositions include non-aqueous carriers such as propylene glycol, polyethylene glycol, vegetable oils such as olive oil, and injectable organic esters such as ethyl oleate, and aqueous carriers such as water and alcoholic/aqueous solutions, including saline and buffered media. The pharmaceutical composition may also include preservatives and other additives such as, for example, anti-microbials, anti-oxidants, chelating agents, and the like.

The present invention provides a method of enhancing the cognitive activity of an animal, the method comprising the step of administering to the animal an effective amount of a compound of formula I as defined above or a pharmaceutically acceptable salt or solvate thereof. The present invention also provides a method of stimulating memory capacity in an animal, the method comprising the step of administering to the animal an effective amount of a compound of formula I as defined above or a pharmaceutically acceptable salt or solvate thereof. As used herein, the term "effective amount" refers to an amount effective to yield the desired therapeutic response.

Typically the animal is a human. However, in some embodiments, the animal is a non-human animal, for example a companion animal such as a dog or cat, or a domestic animal such as a horse, pony, donkey, mule, llama, alpaca, pig, cattle or sheep, or a zoo animal such as a non-human primate, a felid, a canid, a bovid or an ungulate.

The effective amount of the compound will depend on the nature of the condition to be treated, the age, weight and underlying state of health of the individual to be treated, the duration of treatment, the nature of concurrent therapy (if any) and the specific formulation employed. The dosage administered will be at the discretion of the attending physician or veterinarian. The effective amount may readily be determined by those skilled in the art by trial and error experimentation, using methods which are well known in the art.

### Examples

The invention is described below in detail by reference to the following non-limiting examples.

### Example 1 - Synthesis

Compounds of formula I in which R is methyl, ethyl, isopropyl or butyl (compounds **(15), (16), (17)** and **(18)** respectively) were prepared as outlined in the reaction Scheme 1 described below. A similar process can be used to prepare the compound of formula I in which R is propyl. In addition to the compounds of formula I (compounds **(15), (16), (17)** and **(18))** a similar compound in which R is benzyl was also prepared (compound **(19)).**

In the following Schemes, Me = methyl, Et = ethyl, iPr = isopropyl, Bu = butyl and Bz = benzyl.

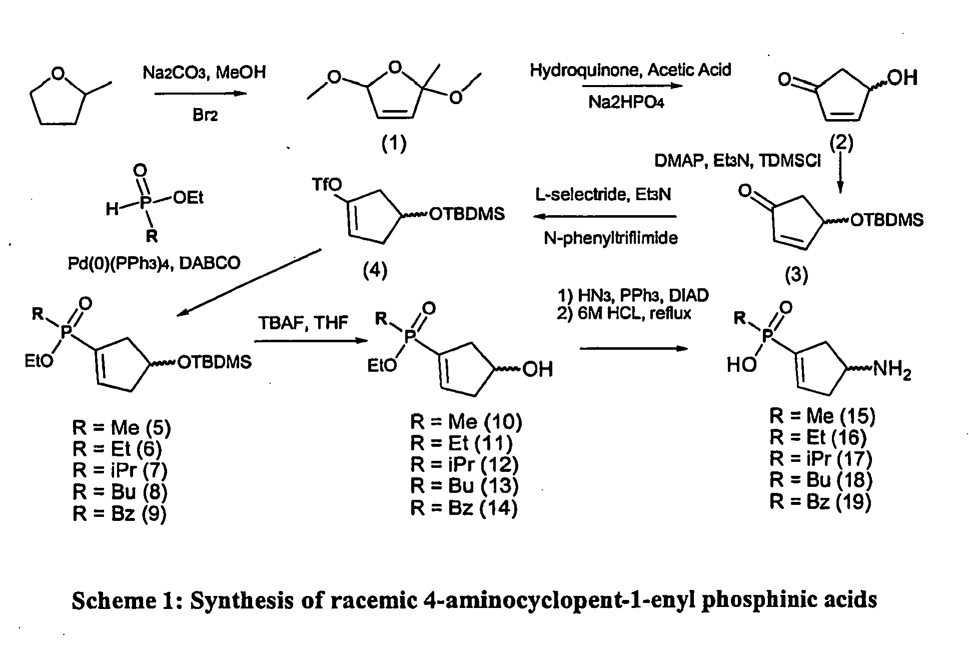

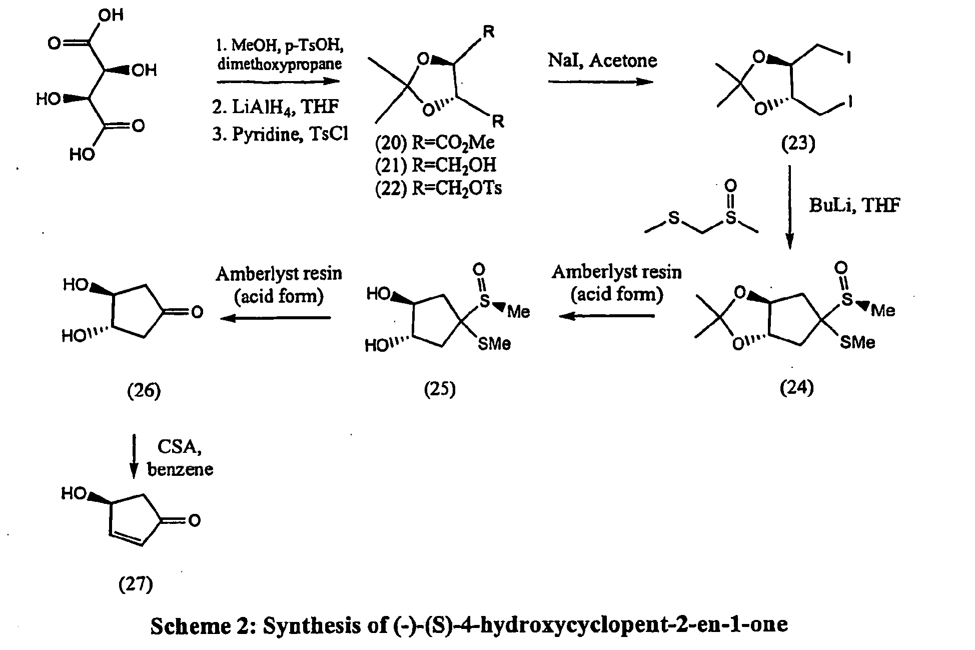

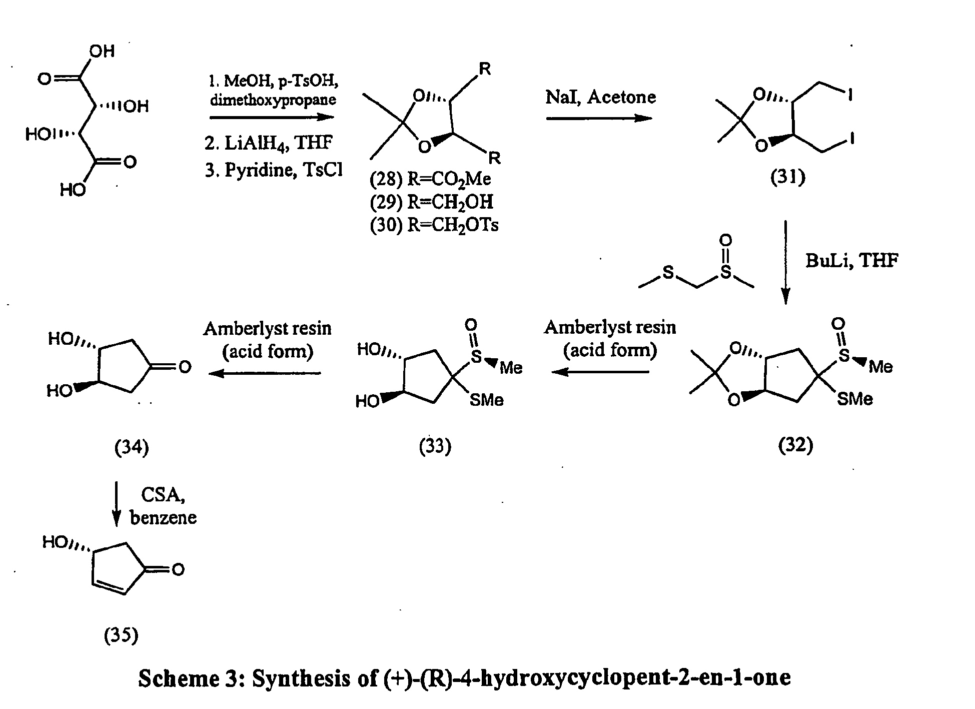

The synthesis of (-)-(S)-hydroxycyclopent-2-en-1-one from L-tartaric acid would allow a person skilled in the art to synthesise optically active 4-aminocyclopent-1-enyl phosphinic acids using the methods shown in Scheme 1. Similarly, the synthesis of (+)-(R)-hydroxycyclopent-2-en-1-one from D-tartaric acid would allow a person skilled in the art to synthesise optically active 4-aminocyclopent-1-enyl phosphinic acids using the methods shown in Scheme 1.

All reagents used in the following Examples were of reagent grade or higher. Tetrahydrofuran (THF) was freshly distilled from sodium benzophenone ketyl and all other solvents were distilled prior to use. ¹H and ¹³C NMR were recorded on a Varian 300M instrument in the indicated solvents. All coupling constants (J values) are given in hertz. Low resolution mass spectra was recorded using either chemical ionization (LRCIMS) or electron impact (LREIMS). High resolution mass spectra was recorded using electron impact only (HREIMS). Optical rotations were measured using a polar polarimeter and a 1dm cell.

The following examples detail the reactions shown in Scheme 2.

### Dimethyl 2,3-O-isopropylidene-L-tartarate (20)

A solution of L-tartaric acid (80g, 533mol), p-toluenesulfonic acid (0.32g, 1.86mmol) and 2,2-dimethoxypropane (127g, 1.2mol) was refluxed gently with stirring for 1.5 hours. Cyclohexane and further 2,2-diemthoxypropane (63.7ml, 0.63mol) was added and azeotropes slowly removed form the head of a vigreaux column. After approximately three days the solution was allowed to cool to room temperature and anhydrous potassium carbonate added to neutralize the catalyst. The solution was then filtered and the solvent removed in-vacuo. The crude product was then distilled at high vacuum to give dimethyl 2,3-O-isopropylidene-L-tartarate (90g, 412mmol 78%) as a yellow oil. ¹H NMR (CDCl₃): δ 4.85 (s, 2H), 3.86 (s, 6H), 1.53 (s, 6H); ¹³C NMR (CDCl₃): δ 172.16, 114.13, 72.25, 53.40, 26.55; LRCIMS: 218.9 (M+1).

### 2,3-O-Isopropylidene-L-threiotol (21)

Lithium aluminium hydride (10g, 267mmol) in anhydrous THF (120ml) was refluxed under nitrogen for thirty minutes with stirring. A solution of dimethyl 2,3-O-isopropylidene-L-tartarate **(20)** (53g, 243mmol) in anhydrous THF (200ml) was added dropwise with stirring. The heat of addition resulted in a gentle reflux. The solution was then refluxed for an additional 3 hours, after which the solution was cooled to 0°C. Water (8.4ml), sodium hydroxide (4M, 8.4ml) and water (32ml) were consecutively added to the solution. The resulting precipitate was removed from the solution by filtration and extracted with diethyl ether (soxhlet). The combined extracts were dried and evaporated at reduced pressure to give 2,3-O-isopropylidene-L-threiotol (35g, 216mmol, 89%). ¹H NMR (CDCl₃): δ 3.98 (m, 2H), 3.77 (m, 4H), 3.64 (s, 2H), 1.45 (s, 6H); ¹³C NMR (CDCl₃): δ 109.53, 78.24, 62.25, 27.28; LREIMS: 162.1 (M⁻). The crude product can be used in the next step.

### 1,4-Ditosyl-2,3-O-isopropylidene-L-threitol (22)

A solution of 2,3-O-isopropylidene-L-threiotol **(21)** (35g, 216mmol) in dry pyridine (500ml) was cooled to -10°C. Finely powdered p-tosylchloride (86g, 453mmol) was added in one portion and the solution stirred until homogenous. The solution was then held at 0°C overnight without stirring. The product was then crystallized from the mixture by the slow addition of water at 0°C. Once visible crystals had begun to form, water was added more rapidly until 500mls of water had been added over three hours at 0°C. Crystallization was then allowed to continue for an additional 1.5 hours at which stage the product was isolated by filtration and washed on the frit with water. The product was then re-crystallized from ethanol to give 1,4-ditosyl-2,3-O-isopropylidene-L-threitol (65g, 138mmol, 68%) as colourless crystals. ¹H NMR (CDCl₃): δ 7.82 (d, J=8.2 , 2H), 7.40 (d, J=8.1 , 2H), 4.13 (m, 4H), 4.04 (m, 2H), 2.48 (s, 6H), 1.33 (s, 6); ¹³C NMR (CDCl₃): δ 148.48, 132.70, 130.24, 128.26, 111.09, 75.32, 68.66, 26.99, 21.94; LRCIMS: 470.9 (M+1).

### 1,4-Dideoxy-1,4-diiodo-2,3-O-isopropylidene-L-threitol (23)

To a solution of 1,4-ditosyl-2,3-O-isopropylidene-L-threitol **(22)** (65g, 138mmol) in dry acetone (800ml) was added sodium iodide (85g, 553mmol) and the mixture refluxed overnight. The solution was then cooled to room temperature, filtered and the filtrate evaporated at reduced pressure. The residue was then dissolved in ethyl acetate (100ml), filtered, washed with water, sodium thiosulphate (5%), brine and then evaporated at reduced pressure. The crude product was then purified via flash chromatography on silica gel (hexane 5% ethylacetate) to give 1,4-dideoxy-1,4-diiodo-2,3-O-isopropylidene-L-threitol (40.5g, 105mmol, 75.8%) as a yellow oil. ¹H NMR (CDCl₃): δ 3.89 (m, 2H), 3.40 (m, 4H), 1.50 (s, 6); ¹³C NMR (CDCl₃): δ 110.38,80.84,27.94, 6.94; LRCIMS: 382.6 (M+1).

### (3S,4S)-3,4-O-Isopropylidene-threitolcyclopentanonedimethyldithioketal-S-oxide (24)

To a solution of methyl methylsulfinyl sulfide (5g, 40mmol) in anhydrous THF (150ml) at -70°C under nitrogen was added butyl lithium (1.6M solution in hexane, 20ml), dropwise over 15 minutes. The solution was stirred at -70°C for one hour before being allowed to warm slowly to room temperature and stirred for an additional one hour. The solution was then re-cooled to -70°C and 1,4-dideoxy-1,4-diiodo-2,3-O-isopropylidene-L-threitol **(23)** was added dropwise over 20 minutes. The solution was then allowed to warm slowly to room temperature, heated to 25°C and stirred for an additional three days under nitrogen. Saturated ammonium chloride (100ml) containing triethylamine (1ml) was then added with stirring. The aqueous phase was then separated and extracted with dichloromethane (2x50ml). The combined organic extracts were evaporated at reduced pressure and purified via flash chromatography on a silica gel column that was pre-treated with hexane containing 1% triethylamine and eluted with ethyl acetate containing 20% acetone and 1% triethylamine to give (3S,4S)-3,4-O-isopropylidene-threitolcyclopentanonedimethyldithioketal S-oxide (2.7g, 11mmol, 74%) as a colourless oil that crystallized upon standing. ¹H NMR (CD₃OD major isomer): δ 4.17 (m, 1H), 2.74 (s, 3H), 2.72 (dd, 2H, J=6.7, J=12.9), 2.34 (s, 3H), 1.85 (dd, 2H, J=5.9, J=11.9), 1.50 (s, 6); ¹H NMR (CD₃OD, minor isomer): δ 3.90 (m, 1H), 2.68 (s, 3H), 2.54 (dd, 2H, J=6.9, J=12.4), 2.26 (s, 3H), 2.11 (dd, 2H, J=6.1, J=12.0), 1.53 (s, 6H); ¹³C NMR (CD₃OD): δ 120.61, 81.49, 72.16, 34.25, 31.67, 26.23, 12.31. Instability prevented MS analysis.

### (3S,4S)-3,4-Dihydroxy-threitolcyclopentanonedimethyldithioketal-S-oxide (25)

To a solution of (3S,4S)-3,4-O-isopropylidene-threitolcyclopentanonedimethyldithioketal-S-oxide **(24)** (1.9g) in methanol (20ml) and water (1ml) was added amberlyst resin (acid form, 0.3g). The solution was stirred for 15 minutes at room temperature and monitored by TLC. Upon consumption of the starting material, the solution was filtered to remove the resin and neutralized with sodium bicarbonate. The solution was then filtered through florisil eluted with dichloromethane containing 10% methanol. The solvent was then removed at reduced pressure and the residue purified via flash chromatography on silica gel eluted with dichloromethane containing 10% methanol to give (3S,4S)-3,4-dihydroxy-threitolcyclopentanonedimethyldithioketal-S-oxide (1.1g, 69%) as a colourless oil.

Minor Isomer ¹H NMR (CD30D) δ 4.28 (m, 1H), 4.03 (m, 1H), 2.78 (dd, 1H, J=7.1, J=15.1), 2.70 (s, 3H), 2.59 (dd, 1H, J=7.7, J=14.6), 2.27 (s, 3H), 2.14 (dd, 1H, J=8.5, J=15.8), 1.75 (dd, 1H, J=5.8, J=13.5); ¹³C NMR (CD₃OD) δ 76.32, 73.91, 69.88, 43.92, 41.03, 20.97, 12.23
Major Isomer ¹H NMR (CD₃OD) δ 4.15 (m, 1H), 3.93 (dd, 1H, J=6.3, J=8.3), 2.67 (s, 3H), 2.50 (dd, 1H, J=6.2, J=13.3), 2.34 (dd, 1H, J=4.3, J=11.1), 2.24 (s, 3H), 2.07 (dd, 1H, J=6.4, J=13.8), 1.96 (dd, 1H, J=6.7, J=13.9); ¹³C NMR (CD₃OD) δ 76.32, 73.91, 69.88, 43.92, 41.03, 20.97, 12.23. Instability prevented MS analysis.

### (3S,4S)-3,4-Dihydroxycyclopentanone (26)

To a solution of (3S,4S)-3,4-dihydroxy-threitolcyclopentanonedimethyldithioketal-S-oxide **(25)** (1.1g) in methanol (10ml) and water (0.5ml) was added amberlyst resin (acid form, 2g). The solution was stirred for 3 hours at room temperature. Upon consumption of the starting material, the solution was filtered to remove the resin and neutralized with sodium bicarbonate. The solution was then filtered through florisil eluted with dichloromethane containing 10% methanol. The solvent was then removed at reduced pressure, the residue redissolved in benzene (20ml) and dried in-vacuo to remove all water. The residue was then purified via flash chromatography on silica gel eluted with dichloromethane containing 10% methanol to give (3S,4S)-3,4-dihydroxycyclopentanone (0.4g, 3mmol, 66%) as a colourless oil. ¹H NMR (*d*₆-acetone): δ 4.42 (d, 2H, J=2.8), 4.33 (m, 2H), 2.55 (dd, 2H, J=4.8, J=17.2), 2.05 (d, 2H, J=18.0); ¹³C NMR (*d*₆-acetone): δ 205.76, 74.21, 44.19; LRCIMS: 116.9 (M+1).

### (-)-(S)-4-Hydroxycyclopent-2-en-1-one (27)

A solution of (3S,4S)-3,4-dihydroxycyclopentanone **(26)** (0.4g, 3.4mmol) and a catalytic quantity of *dl*-10-camphorsulfonic acid (CSA) (10mg, 0.043mmol) in benzene (70 ml) was heated to reflux. Water was removed from the solution using a dean stark apparatus. After approximately three hours the solution was concentrated to approximately 40 ml by removing solvent from the dean stark apparatus. The solution was then refluxed for a further 4 hours before having the solvent removed in-vacuo and purified via flash chromatography on silica gel eluted with dichloromethane containing 10% methanol to give (-)-(S)-4-hydroxycyclopent-2-en-1-one (0.17g, 1.7mmol, 53%) as a yellow oil. ¹H NMR (CDCl₃):7.61 (dd, 1H, J=2.3, J=5.7), 6.25 (dd, 1H, J=1.3, J=5.7), 5.07 (m, 1H), 2.80 (dd, 1H, J=6.1, J=18.5), 2.30 (dd, 1H, J=2.2, J=18.5); ¹³C-NMR (CDCl₃):δ 207.8, 164.4, 134.5, 70.0, 44.1; [α]_{D} = -89.7 (c=1.2) ee > 99%; LRCIMS: 99.0 (M+1).

As shown in Scheme 3, (+)-(R)-4-hydroxycyclopent-2-en-1-one **(35)** may be synthesized using D-tartaric acid as the starting material and source of chirality in a similar manner to that for (-)-(S)-4-hydroxycyclopent-2-en-1-one, as described above. The characteristics of each of the compounds shown in Scheme 3 (compounds **(28)** to **(35))** are detailed below.

### Dimethyl 2,3-O-isopropylidene-D-tartarate (28)

(99%); ¹H NMR (CDCl₃): δ 4.81 (s, 2H), 3.83 (s, 6H), 1.50 (s, 6); ¹³C NMR(CDCl₃):δ 170.28, 114.08, 77.21, 53.03, 26.53; GCCIMS: single peak @ 5.71 minutes m/z: 218.9 (M+1).

### 2,3-O-Isopropylidene-D-threiotol (29)

(89%); ¹H NMR (CDCl₃): δ 4.05 (m, 2H), 3.80 (m, 4H), 1.47 (s, 6H); ¹³C NMR (CDCl₃):δ 109.53, 78.14, 62.03, 27.29; LREIMS: 162.9 (M).

### 1,4-Ditosyl-2,3-O-isopropylidene-D-threitol (30)

(93%); ¹H NMR (CDCl₃): δ 7.82 (d, 2H, J=8.4), 7.40 (d, 2H, J=8.1), 4.13 (m, 4H), 4.05 (m, 2H), 2.49 (s, 6H), 1.33 (s, 6H); ¹³C NMR (CDCl₃): δ 145.50, 132.70, 130.24, 128.26, 111.09, 75.30, 68.66, 26.98, 21.93; LRCIMS: 470.9 (M+1).

### 1,4-Dideoxy-1,4-diiodo-2,3-O-isopropylidene-D-threitol (31)

(99%); ¹H NMR (CDCl₃): δ 3.88 (m, 2H), 3.40 (m, 4H), 1.50 (s, 6H); ¹³C NMR (CDCl₃):δ 110.37, 80.51, 27.95, 6.59; GCCIMS: single peak @ 7.83 minutes m/z: 382.5 (M+1).

### (3R,4R)-3,4-O-Isopropylidene-threitolcyclopentanonedimethyldithioketal-S-oxide (32)

(70%); ¹H NMR (CD₃OD, major isomer): δ 4.17 (m, 1H), 2.74 (s, 3H), 2.72 (dd, 2H, J=6.7, J=12.9), 2.34 (s, 3H), 1.85 (dd, 2H, J=5.9, J=11.9), 1.50 (s, 6H); ¹H NMR (CD₃OD, minor isomer): δ 3.90 (m, 1H), 2.68 (s, 3H), 2.54 (dd, 2H, J=6.9, J=12.4), 2.26 (s, 3H), 2.11 (dd, 2H, J=6.1, J=12.0), 1.53 (s, 6H); ¹³C NMR (CD₃OD): δ 120.61, 81.49, 72.16, 34.25, 31.67, 26.23, 12.31.

### (3R,4R)-3,4-Dihydroxy-threitolcyclopentanonedimethyldithioketal-S-oxide (33)

(90%); ¹H NMR (minor isomer, CD₃OD) δ 4.28 (m, 1H), 4.03 (m, 1H), 2.78 (dd, 1H, J=7.1, J=15.1), 2.70 (s, 3H), 2.59 (dd, 1H, J=7.7, J=14.6), 2.27 (s, 3H), 2.14 (dd, 1H, J=8.5, J=15.8), 1.75 (dd, 1H, J=5.8, J=13.5); ¹³C NMR (CD₃OD) δ 76.32, 73.91, 69.88, 43.92, 41.03, 20.97, 12.23.
¹H NMR (major isomer, CD₃OD) δ 4.15 (m, 1H), 3.93 (dd, 1H, J=6.3, J=8.3), 2.67 (s, 3H), 2.50 (dd, 1H, J=6.2, J=13.3), 2.34 (dd, 1H, J=4.3, J=11.1), 2.24 (s, 3H), 2.07 (dd, 1H, J=6.4, J=13.8), 1.96 (dd, 1H, J=6.7, J=13.9); ¹³C NMR (CD₃OD) δ 76.32, 73.91, 69.88, 43.92, 41.03, 20.97, 12.23.

### (3R,4R)-3,4-Dihydroxycyclopentanone (34)

(75%); ¹H NMR (CD₃OD): δ 4.28 (dt, 2H, J=1.6, J=4.9), 3.34 (dt, 2H, J=1.7, J=3.2), 2.62 (dd, 2H, J=5.8, J=18.0), 2.13 (d, 2H, J=17.0);
¹³C NMR (CD₃OD): δ 217.62, 73.91,43.91; LRCIMS: 116.9 (M+1).

### (+)-(R)-4-Hydroxycyclopent-2-en-1-one (35)

(79%); ¹H NMR (CDCl₃): δ 7.61 (dd, 1H, J=2.3, J=5.6), 6.25 (dd, 1H, J=1.3, J=5.7), 5.08 (m, 1H), 2.80 (dd, 1H, J=6.1, J=18.5), 2.30 (dd, 1H, J=2.2, J=18.5) ¹³C-NMR (CDCl₃): δ 207.8, 164.4, 134.5, 70.0, 44.1; [α]_{D} =+88.31 ee - 98%; LRCIMS: 99.0 (M+1).

The synthesis of the compounds shown in Scheme 1 will now be described.

### 2,5-Dihydro-2,5-dimethoxy-2-methylfuran (1)

To a mechanically stirred mixture of 2-methylfuran (18.2g, 222mol), Na₂CO₃ (47.0g, 443mol, 2.02 equiv) and 170ml of methanol at -55°C was added a solution of bromine (35.4g, 222mol, 1 equiv) in CH₂Cl₂ (12ml) over a one hour period. The reaction mixture was allowed to warm to 10°C over three hours, then filtered through celite. The filtrate was then mixed with brine (200ml) and extracted with CH₂Cl₂ (1 x 200ml portion, 5 x 100ml portions). The combined organic fractions were dried, filtered and concentrated in vacuo to give 2,5-dihydro-2,5-dimethoxy-2-methylfuran (24.9g 145mmol, 79%) as a yellow oil. ¹H NMR (major isomer, CDCl₃): δ 5.9-6.0 (m, 2H), 5.47 (s, 1H), 3.49 (s, 3H), 3.19 (s, 3H), 1.50 (s, 3H); ¹H NMR (minor isomer, CDCl₃): δ 5.9-6.0 (m, 2H), 5.74 (s, 1H), 3.41 (s, 3H), 3.10 (s, 3H), 1.58 (s, 3H); ¹³C NMR δ 134.4, 134.0, 130.1, 129.7, 112.7, 111.5, 107.7, 106.7, 55.5, 54.2, 49.9, 49.3, 26.1, 25.7.

### (±)-4-Hydroxycyclopent-2-en-1-one (2)

To a stirred deoxygenated solution of 2,5-dihydro-2,5-dimethoxy-2-methylfuran (1) (10g, 69.4mmol) and hydroquinone (50mg) in water (278ml) at 0°C was added acetic acid (0.84g, 14mmol, 0.2 equiv). After one hour a solution of Na₂HPO₄.7H₂O (18.6g, 69.4mmol, 1 equiv) in water (60ml) was added and the mixture heated to 60°C for two hours. Non-polar side products were removed by washing with hexanes (100ml) and the aqueous layer extracted with 1-butanol (7 x 100ml). The combined fractions were concentrated under reduced pressure to give a brown oil which was purified by flash chromatography using ethyl acetate:hexane (2:1) as the eluant on silica gel to give (±)-4-hydroxycyclopent-2-en-1-one as a yellow oil (42%). ¹H-NMR (CDCl₃): δ 7.63 (dd, 1H, J=2.4, 5.6), 6.21 (dd, 1H, J=1.3, 5.7), 4.25 (d, 1H), 5.02-5.05 (m, 1H), 2.76 (dd, 1H, J=6.4,18.5), 2.27 (dd, 1H, J=2.1, 18.7); ¹³C-NMR (CDCl₃): δ 207.8, 164.4, 134.5, 70.0,44.1; LRCIMS: 99.0 (M+1).

The following reactions of Scheme 1 are described using (±)-4-hydroxycyclopent-2-en-1-one (**2**) as a reagent. However, as would clearly be understood by a person skilled in the art, these reactions could also be performed using either (+)-(R)-4-hydroxycyclopent-2-en-1-one (**35**) or (-)-(S)-4-hydroxycyclopent-2-en-1-one (**27**) to obtain an enantiopure product. The characteristics of the enantiopure compounds synthesised by such methods are listed following the description of the synthesis of the respective compounds shown in Scheme 1.

### (±)-4-tert-Butyldimethylsiloxycyclopent-2-en-1-one (3)

To a stirred solution of 4-hydroxycylcopent-2-en-1-one (**2**) (1.66g, 16.88 mmol), dimethylaminopyridine (0.206g, 10 mol%) in dry dichloromethane (33ml) at 0°C under inert atmosphere, a solution of tert-butyldimethylsilylchloride (3.13g, 20.68 mmol, 1.2 equiv) in dry dichloromethane (11ml) was added dropwise over ten minutes. The solution was allowed to warm to room temperature and stirred for 3 hours, after which time 100ml of deionized water was added. The organic layer was separated, the aqueous phase extracted with dichloromethane (3 x 50ml) and the combined organic fractions dried with magnesium sulphate and concentrated in vacuo. The resulting oil was filtered using a short column of silica gel eluted with 10% ethyl acetate in petroleum ether. The fractions containing the product were concentrated in vacuo and then distilled under high vacuum to give (±)-4-tert-butyldimethylsiloxycyclopent-2-en-1-one as a colourless oil (2.15g, 14.35mmol, 85%). ¹H NMR (CDCl₃): δ 7.33 (dd, 1H, J=2.3, J=5.7), 6.06 (dd, 1H, J=1.3, J=5.6), 4.86 (m, 1H), 2.59 (dd, 1H, J=5.9, J=18.2), 2.12 (dd, 1H, J=2.3, J=18.2), 0.79 (s, 1H), 0.01 (d, 1H, J=3.6); ¹³C NMR (CDCl₃): δ 164, 135, 71.3, 45.5, 40.5, 25.5, 18.5, -4.43; GCMS:single peak @ 5.96minutes m/z: 213.0 (M+1).

### (-)-(S)-4-tert-Butyldimethylsiloxycyclopent-2-en-1-one

¹H NMR (CDCl₃): δ 7.33 (dd, 1H, J=2.3, J=5.7), 6.06 (dd, 1H, J=1.3, J=5.6), 4.86 (m, 1H), 2.59 (dd, 1H, J=5.9, J=18.2), 2.12 (dd, 1H, J=2.3, J=18.2), 0.79 (s, 1H), 0.01 (d, 1H, J=3.6); ¹³C NMR (CDCl₃): δ 164.08,134.69, 71.12,45.23,40.51,25.99,18.34,-4.44; GCMS: single peak @ 5.95 minutes m/z: 212.9 (M+1); [α]_{D} = -64.7 (c=1.3, MeOH).

### (+)-(R)-4-tert-Butyldimethylsiloxycyclopent-2-en-1-one

¹H NMR (CDCl₃): δ, 7.48 (dd, 1H, J=2.3, J=5.7), 6.21 (dd, 1H, J=1.3, J=5.7), 5.02 (m, 1H), 2.74 (dd, 1H, J=6.0, J=18.2), 2.27 (dd, 1H, J=2.3, J=18.2), 0.94 (s, 9H), 0.16 (d, 6H, J=3.5); ¹³C NMR (CDCl₃): δ 164.18, 134.62, 71.03, 45.27, 40.51, 25.93, 18.31, - 4.46; GCMS:213.0 (M+1); [α]_{D} =+59.8 (c=0.5, MeOH).

### (±)-4-tert-Butyldimethylsiloxycyclopent-1-en-1-yl-tritluoromethanesulfonate (4)

To a solution of L-selectride (1M in THF, 4.7ml, 4.7mmol) in anhydrous THF (35ml) at -78°C under nitrogen was added drop wise a solution of 4-tert-butyldimethylsiloxycyclopent-2-en-1-one (**3**) (1.0g, 4.7mmol) and triethylamine (0.2ml) in THF (15ml) over 30 minutes. After an additional 30 minutes, n-phenyl triflimide (1.5g, 4.1mmol) was added in two portions. The resulting solution gradually warmed to room temperature overnight, the solvent removed in vacuo and the residue partitioned between saturated sodium bicarbonate solution (50ml) and petroleum ether (100ml) overnight. The aqueous layer was extracted with petroleum ether (2 x 25ml) and the combined organic fractions were washed with brine, dried with magnesium sulphate and concentrated in vacuo. The residue was then purified via flash chromatography using 1% triethylamine and 1%ethylacetate in petroleum ether as the eluent on silica gel to give (±)-4-tert-butyldimethylsiloxycyclopent-1-en-1-yl-trifluoromethanesulfonate as a colourless oil (1.59g, 98%). ¹H NMR (CDCl₃): δ 5.58 (m, 1H), 4.60 (tt, 1H, J=3.9, J=7.3), 2.86 (dddd, 1H, J=2.1, J=3.4, J=7.3, J=16.3), 2.73 (ddd, 1H, J=1.2, J=7.1, J=16.5), 2.56 (dtd, 1H, J=2.0, J=4.0, J=16.3), 2.37 (m, 1H), 0.92 (s, 9H), 0.10 (s, 6H); ¹³C NMR (CDCl₃): δ 146.66, 118.78 (q, J=320.8), 115.59, 69.81, 41.46, 39.29, 25.97, 18.26, -4.63; GCMS:single peak @ 6.38minutes m/z: 346.8 (M+1).

### (+)-(R)-4-tert-Butyldimethylsiloxycyclopent-1-en-1-yl-trifluoromethanesulfonate

¹H NMR (CDCl₃): δ 5.58 (m, 1H), 4.60 (tt, 1H, J=3.9, J=7.3), 2.86 (dddd, 1H, J=2.1, J=3.4, J=7.3, J=16.3), 2.73 (ddd, 1H, J=1.2, J=7.1, J=16.5), 2.56 (dtd, 1H, J=2.0, J=4.0, J=16.3), 2.37 (m, 1H), 0.92 (s, 9H), 0.10 (s, 6H); ¹³C NMR (CDCl₃): δ 146.59, 118.69 (q, J=380.8), 115.38, 69.69, 41.29, 39.16, 25.75, -4.41; GCMS:346.8 (M+1) [α]_{D} = +1.97 (c=1.01, MeOH).

### (-)-(S)-4-tert-Butyldimethylsiloxycyclopent-1-en-1-yl-trifluoromethanesulfonate

¹H NMR (CDCl₃): δ 5.58 (m, 1H), 4.60 (tt, 1H, J=3.8, J=7.4), 2.86 (dddd, 1H, J=2.1, J=3.5, J=7.3, J=16.3), 2.73 (dd, 1H, J=7.1, J=16.5), 2.55 (qdd, 1H, J=2.0, J=4.0, J=16.3), 2.37 (d, 1H, J=16.3), 0.92 (s, 9H), 0.10 (s, 6H); ¹³C NMR (CDCl₃): δ 146.65, 118.78 (q, J=320.6), 115.60, 69.81, 41.46, 39.29, 25.98, 18.26,.-4.62; GCCIMS: rt single peak @ 6.40 minutes m/z: 346.7 (M+1); [α]_{D} = -1.92 (c=1.3, MeOH).

The synthesis of various phosphinates to be used in the synthesis of compounds (**5**) to (**9**) from compound (**4**), as shown in Schedule 1, will now be described.

### Ethyl 1,1-diethoxyethylphosphinate

Aqueous phosphinic acid was concentrated in vacuo at a temperature of less than 40°C to give at least 95% pure acid, which slowly crystallized upon standing. Triethyl orthoactetate (200ml) was cooled to 5°C under nitrogen, and boron trifluoride diethyletherate (5ml) was added slowly. The mixture was further cooled to -5°C and the phosphinic acid (16.3g) was added slowly. Temperature was precisely maintained at -5°C during the addition. The mixture was then warmed to room temperature (r.t.) over 3 hours. Dihydrogen sodium phosphate solution (1.2M, 50ml) was then added in one portion with stirring. The solution was extracted with dichloromethane (2x50ml). The combined organic layers where then washed with water, dried using Mg₂SO₄ and the solvent was removed in vacuo at a temperature of less than 40°C. The product was then used without further purification in the next step (i.e. the reaction with compound (4)). ¹H NMR (CDCl₃): δ 6.95 (d, 1H, J=543.8), 1.50 (d, 3H, J=12.6), 1.39 (t, 3H, J=7.1), 1.24 (dt, 6H, J=2.2, J=4.9), 4.23 (m, 2H), 3.71 (m, 4H); ¹³C NMR (CDCl₃): δ 100.36 (d, PC (OCH₂CH₃)₂CH₃, J_{PC}=150.6), 63.36 (d, POC, ²J_{POC}=7.8), 57.96 (d, PC (OCCH₃)₂, ³J_{PCOC}=8.8), 19.18 (d, POCCH₃, 3J_{POCC}=13.1), 16.58 (d, PC (OCCH₃)₂, ⁴J_{PCOCC}=5.6), 16.43 (d, PCCH₃, ²J_{PCC}=104.4).

MS data was not collected for phosphinates due to the extensive characterization of subsequent coupling products.

### Ethyl 1,1-diethoxyethyl(methyl)phosphinate (alkylation of ethyl 1,1-diethoxyethylphosphinate)

To a solution ofNaH (1.6g, 40mmol) in THF (10ml) was added slowly a solution of ethyl 1,1-diethoxyethylphosphinate (7.0g, 33mmol) in THF (10ml) under N₂ at r.t. The temperature was kept below 30°C during addition. The solution was stirred at r.t. for a further 2 hours at which time ethyl iodide (14.18g, 99mmol) was added slowly. The solution was stirred for an additional 24 hours. The solution was cooled on an ice bath and treated carefully with water. The solution was concentrated in vacuo before being partitioned between water and DCM The DCM layer was dried with Mg₂SO₄ and concentrated in vacuo. The product was distilled at high vacuum. ¹H NMR (CDCl₃): δ 4.16 (m, 2H), 3.70 (m, 4H), 1.50 (d, 3H, J=11.3), 1.47 (d, 3H, J=13.8), 1.33 (t, 3H,

J=7.1), 1.22 (t, 6H, J=7.1); ¹³C NMR (CDCl₃): δ 101.15 (d, PC (OCH₂CH₃)₂CH₃, J_{PC}=143.1), 61.76 (t, POC, ²J_{POC}=5.7), 58.26 (d, PC (OCCH₃)₂, ³J_{PCOC}=5.4), 20.61 (d, POCCH₃, ³J_{POCC}=12.6), 16.89 (d, PC (OCCH₃)₂, ⁴J_{PCOCC}=5.6), 15.66 (d, PCCH₃, ²J_{PCC}=13.9), 11.83 (d, PCH₃, J_{PC}=88.7).

### Ethyl 1,1-diethoxyethyl(ethyl)phosphinate

¹H NMR (CDCl₃): δ 4.19 (m, 2H) 3.70 (m, 4H) 1.76 (m, 2H) 1.50 (d, 3H, J=11.0) 1.32 (m, 3H) 1.21 (m, 9H); ¹³C NMR (CDCl₃): δ 101.47 (d, PC (OCH₂CH₃)₂CH₃, J_{PC}=137.5), 61.62 (d, POC, ²J_{POC}=6.5), 58.30 (d, PC (OCCH₃)₂, ³J_{PCOC}=4.7), 26.31 (d, PCH₂CH₃, J_{PC}=40.4), 20.79 (d, POCCH₃, ³J_{POC}=12.4), 16.70 (d, PC (OCCH₃)₂, ⁴J_{PCOCC}=6.0), 15.65 (d, PCCH₃, ²J_{PCC}=18.8), 13.81 (d, PCH₂CH₃, ²J_{PCC}=5.9).

### Ethyl 1,1-diethoxyethyl(isopropyl)phosphinate

¹H NMR (CDCl₃): δ 4.20 (m, 2H), 3.72 (m, 4H), 2.00 (m, 1H), 1.52 (d, 3H, J=10.8), 1.31 (t, 6H, J=7.1), 1.20 (m, 9H); ¹³C NMR (CDCl₃): δ 102.00 (d, PC (OCH₂CH₃)₂CH₃, J_{PC}=132.3), 61.57 (d, POC, ²J_{POC}=7.1), 58.27 (d, PC (OCCH₃)₂, ³J_{PCOC}=4.5), 26.12 (d, PC (CH₃)₂, J_{PC}=86.5), 20.99 (d, POCCH₃, ³J_{POCC}=12.4), 16.93 (d, PC (OCCH₃)₂, ⁴J_{PCOCC}=5.1), 15.93 (d, PC (CH₃)₂, ²J_{PCC}=5.3), 15.62 (d, PCCH₃, ²J_{PCC}=18.4).

### Ethyl 1,1-diethoxyethyl(butyl)phosphinate

¹H NMR (CDCl₃): δ 4.19 (m, 2H), 3.71 (m, 4H), 1.72 (m, 4H), 1.51 (d, 3H, J=11.1), 1.42 (m, 2H), 1.33 (t, 3H, J=7.1), 1.22 (t, 6H, J=7.1), 0.94 (t, 3H, J=7.3); ¹³C-NMR (CDCl₃): δ 102.34 (d, PC (OCH₂CH₃)₂CH₃, J_{PC}=131.1), 62.84 (d, POC, ³J_{POC}=7.0), 57.21 (d, PC (OCCH₃)₂, ³J_{PCOC}=5.1), 28.48 (d, PCH₂ (CH₂)₂CH₃, J_{PC}=93.8), 23.75 (d, PCH₂CH₂CH₂CH₃, ²J_{PCC}=16.1), 22.92 (d, P(CH₂)₂CH₂CH₃, ³J_{PCCC}=3.0), 18.60 (d, POCH₂CH₃, ³J_{POCC}=13.4), 16.83 (d, PC (OCCH₃)₂, ⁴J_{PCOCC}=4.3), 15.31 (d, PCCH₃, ²J_{PCC}=17.7), 13.76 (P(CH₂)₃CH₃).

### Ethyl 1,1-diethoxyethyl(phenyl)phosphinate

¹H NMR (CDCl₃): δ 7.36 (m, 5H), 4.12 (m, 2H), 3.70 (m, 4H), 1.50 (d, 3H, J=11.2), 1.25 (m, 6H), 1.21 (t, 3H, J=7.1).

### Ethyl methylphosphinate (hydrolysis of alkyl(1,1-diethoxyethyl)phosphinates)

To a solution of ethyl 1,1-diethoxyethyl(methyl)phosphinate (3.7g, 17mmol) in DCM: 10% EtOH (60ml), chlorotrimtheylsilane (2.51g, 23.1mmol) was added dropwise at r.t. The solution was then stirred for 24hours at r.t. before the volatile components were removed in vacuo. The product was used in the next step (i.e. the reaction with compound (4)) without further purification. ¹H NMR (CDCl₃): δ 7.28 (d, 1H, J=552.6), 4.14 (m, 2H), 1.58 (dd, 3H, J=1.8, J=15.3), 1.36 (t, 3H, J=7.1); ¹³C NMR (CDCl₃): δ 62.44 (dd, POC, ²J_{POC}=6.3, J=61.7), 16.51 (d, POCCH₃, ³J_{POCC}=9.4), 11.29 (d, PCH₃, J_{PC}=144.3).

### Ethyl ethylphosphinate

¹H NMR (CDCl₃): δ 7.08 (dt, 1H, J=1.9, J=533.7), 4.16 (m, 2H), 1.83 (m, 2H), 1.37 (t, 3H, J=7.1), 1.17 (td, 3H, J=7.8, J=21.5); ¹³C NMR (CDCl₃): δ 62.92 (d, POC, ³J_{POC}=7.1), 21.83 (d, PCH₂CH₃, J_{PC}=94.7), 16.47 (d, POCCH₃, ³J_{POCC}=6.1), 4.85 (d, PCH₂CH₃, ²J_{PCC}=4.0).

### Ethyl isopropylphosphinate

¹H NMR (CDCl₃): δ 6.88 (dd, 1H, J=1.3, J=521.6), 4.15 (m, 2H), 1.92 (dtt, 1H, J=1.0, J=7.1, J=14.6), 1.36 (t, 3H, J=7.0), 1.17 (ddd, 1H, J=1.1, J=7.2, J=19.7); ¹³C NMR (CDCl₃): δ 63.39 (d, POC, ²J_{POC}=7.6), 27.26 (d, PC (CH₃)₂, J_{PC}=95.3), 16.44 (d,

POCCH₃, ³J_{POCC}=5.9), 14.26 (dd, PC(CH₃)₂, J=1.6, J=11.4).

### Ethyl butylphosphinate

¹H NMR (CDCl₃): δ 7.11 (d, 1H, J=534.7), 4.14 (m, 2H), 1.81 (m, 2H), 1.57 (m, 2H), 1.43 (m, 2H), 1.27 (t, 3H, J=7.1), 0.92 (t, 3H, J=7.2); ¹³C NMR (CDCl₃): δ 62.84 (d, POC, ²J_{POC}=7.0), 28.48 (d, PCH₂ (CH₂)₂CH₃, J=_{PC}93.8), 23.75 (d, PCH₂CH₂CH₂CH₃, ²J_{PCC}=16.1), 22.92 (d, P(CH₂)₂*C*H₂CH₃, ³J_{PCCC}=3.0), 16.60 (d, POCH₂CH₃, ³J_{POCC}=13.4), 13.76 (P(CH₂)₃CH₃).

### Ethyl phenylphosphinate

¹H NMR (CDCl₃): δ 7.34 (m, 5H), 7.10 (d, 1H, J=551.0), 4.11 (m, 2H), 1.27 (t, 3H, J=7.1).

### (±)-Ethyl(4-tert-butyldimethylsiloxycyclopent-1-enyl)methylphosphinate (5) (palladium catalysed C-P bond formation)

To a solution of DABCO (200mg, 2.0mmol, 3 equiv), methylphosphinate (94mg, 0.435mmol, 1.5 equiv) and 4-tert-butyldimethylsiloxycyclopent-1-en-1-yl-trifluoromethanesulfonate (4) (200mg, 0.58 mmol) in toluene (10ml) was added tetrakis- (triphenylphosphine)palladium(0) (17mg, 2.5 mol%). The solution was stirred at room temperature for 18 hours at which time a second portion of tetrakis-(triphenylphosphine)palladium(0) (10mg, 2.5 mol%) was added. The solution was stirred for a further 24 hours, the toluene removed in vacuo and the residue purified via flash chromatography using ethylacetate: 10% ethanol as the eluent on silica gel to give ethyl(4-tert-butyldimethylsiloxycyclopent-1-enyl)methylphosphinate as a colourless oil (143mg, 78%).¹H NMR (CDCl₃): δ 6.62 (ddm, 1H, J=10.8, J=17.8), 4.63 (m, 1H), 4.10 (m, 2H), 2.72 (m, 2H), 2.45 (m, 2H), 1.49 (d, 3H, J=17.5), 1.34 (t, 3H, J=7.1), 0.88 (d, 9H, J=2.6), 0.07 (d, 6H, J=1.3); ¹³C NMR (CDCl₃): δ 145.44 and 144.83 (2d from 2 diastereomers, C (2), ²J_{PCC}=11.5), ²J_{PCC}=10.63) 134.28 (d, C (1), J_{PC}=124.3), 72.99 and 72.94 (2d from 2 diastereomers, C (4), ³J_{PCCC}=11.6, ³J_{PCCC}=11.4), 61.77 (d, POCH₂CH₃, ²J_{POC}=6.3), 44.48 and 44.43 (2d from 2 diastereomers, C (5), ²J_{PCC}=16.3, ²J_{PCC}=15.7), 43.33 (d, C (3), ³J_{PCCC}=12.1), 25.92 (SiC (CH₃)₃), 18.25 (SiC (CH₃)₃), 16.73 and 16.62 (2d from 2 diastereomers, POCH₂*C*H₃, ³J_{POCC}=6.2, ³J_{POC}=6.7), 11.40 (d, PCH₃, J_{CP}=144.4), -4.55 (Si (CH₃)₂); HREIMS: for C₁₄H₂₉O₃PSi calculated (M): 304.1624 observed: 304.1631; GCCIMS: rt = single peak @ 10.07 minutes m/z: 305.1 (M+1).

### (-)-(R)-Ethyl(4-tert-butyldimethylsiloxycyclopent-1-enyl)methylphosphinate

Obtained as a colourless oil (79%).¹H-NMR (CDCl₃): δ 6.62 (ddm, 1H, J=10.8, J=17.8), 4.63 (m, 1H), 4.10 (m, 2H), 2.72 (m, 2H), 2.45 (m, 2H), 1.49 (d, 3H, J=17.5), 1.34 (t, 3H, J=7.1), 0.88 (d, 9H, J=2.6), 0.07 (d, 6H, J=1.3); ¹³C-NMR (CDCl₃): δ 145.44 and 144.83 (2d from 2 diastereomers, C (2), ²J_{PCC}=11.5), ²J_{PCC}10.63) 134.28 (d, C (1), J_{PC}=124.3), 72.99 and 72.94 (2d from 2 diastereomers, C (4), ³J_{PCCC}=11.6, ³J_{PCCC}=11.4), 61.77 (d, PO*C*H₂CH₃, ²J_{POC}=6.3), 44.48 and 44.43 (2d from 2 diastereomers, C (5),²J_{PCC}=16.3, ²J_{PCC}=15.7), 43.33 (d, C (3), ³J_{PCCC}=12.1), 25.92 (SiC (CH₃)₃), 18.25 (Si*C* (CH₃)₃), 16.73 and 16.62 (2d from 2 diastereomers, POCH₂CH₃, ³J_{POCC}=6.2, ³J_{POCC}=6.7), 11.40 (d, PCH₃, J_{CP}=144.4), -4.55 (Si (CH₃)₂); [α]_{D} =5.40; GCCIMS: rt = single peak @ 10.09 minutes m/z: 305.1 (M+1).

### (+)-(S)-Ethyl(4-tert-butyldimethylsiloxycydopent-1-enyl)methylphosphinate

Obtained as a colourless oil (96%). ¹H NMR (CDCl₃): δ 6.62 (tdd, 1H, J=2.0, J=10.9, J=17.8), 4.64 (m, 1H), 4.10 (m, 2H), 2.74 (m, 2H), 2.45 (m, 2H), 1.49 (d, 3H, J=17.5), 1.34 (t, 3H, J=7.1), 0.89 (d, 9H, J=2.6), 0.08 (d, 6H, J=1.4); ¹³C-NMR (CDCl₃): δ 145.44 and 144.83 (2d from 2 diastereomers, C (2), ²J_{PCC=}11.5), ²J_{PCC}=10.63) 134.28 (d, C (1), J_{PC}=124.3), 72.99 and 72.94 (2d from 2 diastereomers, C (4), ³J_{PCCC}=11.6, ³J_{PCC}=11.4), 61.77 (d, PO*C*H₂CH₃, ²J_{POC}=6.3), 44.48 and 44.43 (2d from 2 diastereomers, C (5), ²J_{PCC}=16.3, ²J_{PCC}=15.7), 43.33 (d, C (3), ³J_{PCCC}=12.1), 25.92 (SiC (CH₃)₃), 18.25 (Si*C* (CH₃)₃), 16.73 and 16.62 (2d from 2 diastereomers, POCH₂*C*H₃, ³J_{POCC}=6.2, ³J_{POCC}=6.7), 11.40 (d, PCH₃, J_{CP}=144.4), -4.55 (Si (CH₃)₂); GCCIMS: rt = single peak @ 10.11 minutes m/z: 305.0 (M+1).

A similar procedure was used to prepare compounds **(6)** to **(9).** The characteristics of these compounds, as well as the enantiopure compounds corresponding to compound **(8),** are described below.

### (±)-Ethyl(4-tert-butyldimethylsiloxycyclopent-1-enyl)ethylphosphinate (6)

Obtained as a colourless oil (76%). ¹H NMR (CDCl₃): δ 6.65 (m, 1H), 4.62 (m, 1H), 4.11 (m, 2H), 2.74 (m, 2H), 2.48 (m, 2H), 1.76 (m, 2H), 1.23 (m, 6H), 0.89 (dd, 9H, J=0.5, J=2.0), 0.08 (s, 1H); ¹³C NMR (CDCl₃):δ 145.88 (t, C (2), ²J_{PCC}=10.7), 133.07 (d, C (1), J_{CP}=123.6), 73.05 and 73.04 (2d from 2 diastereomers, C (4), ³J_{PCCC}=10.9, ³J_{PCCC}=10.9), 61.71 (d, PO*C*H₂CH₃, ²J_{POC}=6.5), 44.55 and 44.47 (2d from 2 diastereomers, C (5), ²J_{PCC}=15.3), 43.83 and 43.67 (2d from 2 diastereomers, C (5), ³J_{PCCC}=9.2, ³J_{PCCC}=8.9), 25.91 (SiC (*C*H₃)₃), 21.47 (d, PCH₂CH₃, J_{PC}=102.6), 16.71 (t, POCH₂*C*H₃, J=6.1), 6.79 (d, PCH₂*C*H₃, J=6.8); GCMS: (m/z) 319.1 (M+1); HREIMS: for C₁₅H₃₁O₃PSi calculated (M): 318.1780 observed: 318.1789; GCCIMS: rt: single peak @ 10.53 minutes m/z: 319.1 (M+1).

### (±)-Ethyl(4-tert-butyldimethylsiloxycyclopent-1-enyl)isopropylphosphinate (7)

Obtained as colourless needles (90%). ¹H NMR (CD₃OD): δ 6.71 (dm, 1H, J=9.9), 4.69 (m, 1H), 4.08 (m, 2H), 2.81 (m, 2H), 2.48 (m, 2H), 2.01 (m, 1H), 1.34 (t, 3H, J=7.1), 1.20 (dd, 6H, J=7.2, J=18.7), 0.92 (d, J=0.64, 9H), 0.12 (s, 6H); ¹³C NMR (CD₃OD): δ 147.57 (triplet, C (2), J=9.6), 131.55 and 135.41 (2d from 2 diastereomers, C (1), J_{CP}=121.0, J_{CP}=121.1), 72.88 (d, C (4), ³J_{PCCC}=10.8), 60.91 and 60.87 (2d from 2 diastereomers, POCH₂CH₃, ²J_{POC}=7.0, ²J_{POC}=7.0), 44.41 and 44.34 (2d from 2 diastereomers, C (5), ²J_{PCC}=15.2, ²J_{PCC}=15.3), 44.04 and 43.90 (2d from 2 diastereomers, C (3), ³J_{PCCC}=11.3, ³J_{PCCC}=11.1), 26.81 and 26.64 (2d from 2 diastereomers, PCH (CH₃)₂, J_{CP}=103.2, J_{CP}=102.9), 25.11 (SiC (CH₃)₂), 17.67 (SiC (CH₃)₂), 15.68 (d, POCH₂*C*H₃, ³J_{POCC}=6.0), 13.77 and 13.72 (d, PCH (CH₃)₂, ²J_{PCC}=6.1, ²J_{PCC}=6.2), -5.82 (Si (CH₃)₂); GCCIMS: rt: 2 peaks from 2 diastereomers @ 10.67 and 10.73 minutes m/z: 333.1 (M+1).

### (±)-Ethyl(4-tert-butyldimethylsiloxycyclopent-1-enyl)butylphosphinate (8)

Obtained as colourless needles (94%). ¹H NMR (CD₃OD): δ 6.69 (dm, 1H, J=10.7), 4.69 (m, 1H), 4.06 (m, 2H), 2.82 (m, 2H), 2.47 (m, 2H), 1.82 (m, 2H), 1.54 (m, 4H), 1.36 (t, 3H, J=3.0), 1.29 (t, 3H, J=6.7), 0.92 (d, 9H, J=1.2), 0.12 (s, 6H); ¹³C NMR (CD₃OD): δ 146.60 (overlapping doublets, C (2),²J_{PCC}=11.8), 132.54 (d, C (1), J_{PC}=124.8), 72.82 (d, C (4), ³J_{PCCC}=10.7), 60.79 and 60.74 (2d from 2 diastereomers, PO*C*H₂CH₃, ²J_{POC}=6.5, ²J_{POC}=6.6), 44.46 and 44.41 (2d from 2 diastereomers, C (5), ²J_{PCC}=15.8, ²J_{PCC}=15.8), 43.52 and 43.49 (2d from 2 diastereomers, C (3), ³J_{PCCC}=12.1, ³J_{PCCC}=11.8), 27.17 and 26.92 (2d from 2 diastereomers, P*C*H₂ (CH₂)₂CH₃, J_{PC}=101.4, J_{PC}=100.8), 25.13 (SiC (*C*H₃)₂), 23.59 (d, P (CH₂)₂*C*H₂CH₃, ³J_{PCCC}=23.9), 23.52 (d, PCH₂CH₂CH₂CH₃, ²J_{PCC}=30.3), 17.68 (s, Si*C* (CH₃)₂), 15.67 and 15.59 (2d from 2 diastereomers, POCH₂CH₃, ³J_{POCC}=9.0, ³J_{POCC}=8.7), 12.76 (P (CH₂)₃*C*H₃), -5.84 (Si (*C*H₃)₂); HREIMS: for C₁₇H₃₅O₃PSi calculated (M): 346.2093 observed: 346.2096; GCCIMS: rt: 2 peaks from 2 diastereomers @ 11.45 and 11.49 minutes m/z: 347.1 (M+1).

### (R)-Ethyl(4-tert-butyldimethylsiloxycyclopent-1-enyl)butylphosphinate

Obtained as colourless needles (99%). ¹H-NMR (CD₃OD): δ 6.69 (dm, 1H, J=10.7), 4.69 (m, 1H), 4.06 (m, 2H), 2.82 (m, 2H), 2.47 (m, 2H), 1.82 (m, 2H), 1.54 (m, 4H), 1.36 (t, 3H, J=3.0), 1.29 (t, 3H, J=6.7), 0.92 (d, 9H, J=1.2), 0.12 (s, 6H); ¹³C-NMR (CD₃OD): δ 146.60 (overlapping doublets, C (2),²J_{PCC}=11.8), 132.54 (d, C (1), J_{PC}=124.8), 72.82 (d, C (4), ³J_{PCCC}=10.7), 60.79 and 60.74 (2d from 2 diastereomers, PO*C*H₂CH₃, ²J_{POC}=6.5, ²J_{POC}=6.6), 44.46 and 44.41 (2d from 2 diastereomers, C (5), ²J_{PCC}=1.5.8, ²J_{PCC}=15.8), 43.52 and 43.49 (2d from 2 diastereomers, C (3), ³J_{PCCC}=12.1, ³J_{PCCC}=11.8), 27.17 and 26.92 (2d from 2 diastereomers, P*C*H₂ (CH₂)₂CH₃, J_{PC}=101.4, J_{PC}=100.8), 25.13 (SiC (*C*H₃)₂), 23.59 (d, P (CH₂)₂*C*H₂CH₃, ³J_{PCCC}=23.9), 23.52 (d, PCH₂*C*H₂CH₂CH₃, ²J_{PCC}=30.3), 17.68 (s, SiC (CH₃)₂), 15.67 and 15.59 (2d from 2 diastereomers, POCH₂*C*H₃, ³J_{POCC}-9.0, ³J_{POCC}=8.7), 12.76 (P (CH₂)₃*C*H₃), -5.84 (Si (*C*H₃)₂); [α]D =-2.38; GCCIMS: rt: 2 peaks from 2 diastereomers @ 11.46 and 11.51 minutes m/z: 347.1 (M+1).

### (S)-Ethyl(4-tert-butyldimethylsiloxycyclopent-1-enyl)butylphosphinate

Obtained as colourless needles (92%). ¹H-NMR (CD₃OD): δ 6.69 (dm, 1H, J=10.7), 4.69 (m, 1H), 4.06 (m, 2H), 2.82 (m, 2H), 2.47 (m, 2H), 1.82 (m, 2H), 1.54 (m, 4H), 1.36 (t, 3H, J=3.0), 1.29 (t, 3H, J=6.7), 0.92 (d, 9H, J=1.2), 0.12 (s, 6H); ¹³C-NMR (CD₃OD) δ 146.60 (overlapping doublets, C (2),²J_{PCC}=11.8), 132.54 (d, C (1), J_{PC}=124.8), 72.82 (d, C (4), ³J_{PCCC}=10.7), 60.79 and 60.74 (2d from 2 diastereomers, PO*C*H₂CH₃, ²J_{POC}=6.5, ²J_{POC}=6.6), 44.46 and 44.41 (2d from 2 diastereomers, C (5), ²J_{PCC}=15.8, ²J_{PCC}=15.8), 43.52 and 43.49 (2d from 2 diastereomers, C (3), ³J_{PCCC}=12.1, ³J_{PCCC}=11.8), 27.17 and 26.92 (2d from 2 diastereomers, P*C*H₂ (CH₂)₂CH₃, J_{PC}=101.4, J_{PC}=100.8), 25.13 (SiC (*C*H₃)₂), 23.59 (d, P (CH₂)₂*C*H₂CH₃, ³J_{PCCC}=23.9), 23.52 (d, PCH₂*C*H₂CH₂CH₃, ²J_{PCC}=30.3), 17.68 (s, Si*C* (CH₃)₂), 15.67 and 15.59 (2d from 2 diastereomers, POCH₂*C*H₃, ³J_{PCCC}=O, ³J_{POCC}=8.7), 12.76 (P (CH₂)₃*C*H₃), -5.84 (Si (*C*H₃)₂); GCCIMS: rt: 2 peaks from 2 diastereomers @ 11.46 and 11.51 minutes m/z: 347.1 (M+1)

### (±)-Ethyl(4-tert-butyldimethylsiloxycyclopent-1-enyl)benzylphosphinate (9)

Obtained as a colourless oil. ¹H NMR (CDCl₃): δ 0.09 (s, 6), 0.90 (s, 9) 1.29 (t, 3, J=7.0), 2.45 (m, 2), 2.76 (m, 2), 3.95 (m, 2), 4.65 (m, 1), 6.55 (bd, 1, J=18.0), 7.1-7.5 (m, 5).

### (±)-Ethyl(4-hydroxycyclopent-1-enyl)methylphosphinate (10) (deprotection of tertbutyldimethylsilyl protected alcohol)

To a solution of ethyl(4-tert-butyldimethylsiloxycyclopent-1-enyl)methylphosphinate (5) (350mg, 1.2mmol,1 equiv) under nitrogen was added tert-butylammoniumfluoride (1M in THF, 1.44ml), 1.2 equiv). The solution was stirred at room temperature for 12 hours and the solvent was removed in vacuo. The residue was purified by flash chromatography using ethylacetate:ethanol (75:15) as the eluant on silica gel to give ethyl(4-hydroxycyclopent-1-enyl)methylphosphinate as a yellow oil (208mg, 98%). ¹H NMR (CDCl₃): δ 6.62 (d, 1H, J=10.6), 4.65 (m, 1H), 4.02 (m, 2H), 3.04 (m, 1H), 2.82 (d, 1H, J=16.5), 2.55 (d, 1H, J=18.0), 1.54 (dd, 3H, J=4.3, J=14.5), 1.33 (dt, 3H, J=2.0, J=7.1); ¹³C NMR (CDCl₃): δ 145.63 and 145.51 (2d from 2 diastereomers, C (2), ²J_{PCC}=11.4, ²J_{PCC} =11.8), 133.50 (d, C (1), J_{CP}=131.6), 71.27 (d, C (4), ³J_{PCCC}=11.2), 60.87 and 60.85 (dd, PO*C*H₂CH₃, ²J_{POC}=6.3, ²J_{POC}=6.3), 43.65 (d, C (5), ²J_{PCC}=16.4), 42.53 and 432.45 (2d from 2 diastereomers, C (3), ³J_{PCCC}=12.3, ³J_{PCCC} =12.1), 15.64 (d, POCH₂*C*H₃, ³J_{POCC}=6.6), 12.74 and 12.57 (2d from 2 diastereomers, P*C*H₃, J_{PC}=102.5, J=102.2); LREIMS: 190.1 (M).

### (R)-Ethyl(4-hydroxycyclopent-1-enyl)methylphosphinate

¹HNMR (CDCl₃): δ 6.61 (d, 1H, J=10.6), 4.64 (qdd, 1H, J=2.0, J=3.9, J=8.0), 4.00 (m, 2H), 2.78 (m, 2H), 2.54 (m, 2H), 1.53 (dd, 3H, J=4.5, J=14.5), 1.32 (dt, 3H, J=2.1, J=7.1); ¹³C-NMR (CDCl₃): δ 145.63 and 145.51 (2d from 2 diastereomers, C (2), ²J_{PCC}=11.4, ²J_{PCC} =11.8), 133.50 (d, C (1), J_{CP}=131.6), 71.27 (d, C (4), ³J_{PCCC}=11.2), 60.87 and 60.85 (dd, PO*C*H₂CH₃, ²J_{POC}=6.3, ²J_{POC}=6.3), 43.65 (d, C (5), ²J_{PCC}=16.4), 42.53 and 432.45 (2d from 2 diastereomers, C (3), ³J_{PCCC}=12.3, ³J_{PCCC} =12.1), 15.64 (d, POCH₂*C*H3, ³J_{POCC}=6.6), 12.74 and 12.57 (2d from 2 diastereomers, P*C*H₃, J_{PC}=102.5, J=102.2); LREIMS: 190.1 (M).

### (S)-Ethyl(4-hydroxycyclopent-1-enyl)methylphosphinate

¹H-NMR (CDCl₃): δ 6.61 (d, 1H, J=10.6), 4.64 (qdd, 1H, J=2.0, J=3.9, J=8.0), 4.00 (m, 2H), 2.78 (m, 2H), 2.54 (m, 2H), 1.53 (dd, 3H, J=4.5, J=14.5), 1.32 (dt, 3H, 3=2.1, J=7.1); ¹³C-NMR (CDCl₃): δ 145.63 and 145.51 (2d from 2 diastereomers, C (2), ²J_{PCC}=11.4, ²J_{PCC} =11.8), 133.50 (d, C (1), J_{CP}=131.6), 71.27 (d, C (4), ³J_{PCCC}=11.2), 60.87 and 60.85 (dd, PO*C*H₂CH₃, ²J_{POC}=6.3, ²J_{POC}=6.3), 43.65 (d, C (5), ²J_{PCC}=16.4), 42.53 and 432.45 (2d from 2 diastereomers, C (3), ³J_{PCCC}=12.3, ³J_{PCCC} =12.1), 15.64 (d, POCH₂*C*H₃, ³J_{POCC}=6.6), 12.74 and 12.57 (2d from 2 diastereomers, PCH₃, J_{PC}=102.5, J=102.2); LREIMS: 190.1 (M).

A similar procedure was used to prepare compounds (11) to (14). The characteristics of these compounds, as well as the enantiopure compounds corresponding to compound (13), are described below.

### (±)-Ethyl(4-hydroxycyclopent-1-enyl)ethylphosphinate (11)

¹H NMR (CDCl₃): δ 6.62 (dm, 1H, J=10.0), 4.64 (t, 1H, J=5.5), 4.02 (m, 2H), 2.86 (s, 1H), 2.78 (m, 2H), 2.53 (m, 2H), 1.78 (m, 2H), 1.32 (dt, 3H, J=2.0, J=7.1), 1.13 (dtd, 3H, J=2.2, J=7.7, J=18.6); ¹³C NMR (CDCl₃): δ 145.55 and 145.46 (2d from 2 diastereomers, C (2), ²J_{PCC}=10.3, ²J_{PCC}=10.2), 133.32 (d, C (1), J_{PC}=124.2), 72.00 (d, C (4), ³J_{PCCC}=10.4), 60.64 and 60.61 (2d from 2 diastereomers, PO*C*H₂CH₃, ²J_{POC}=6.4, ²J_{POC} =6.4), 44.39 (d, C (5), ²J_{PCC}=15.6), 43.62 and 43.57 (2d from 2 diastereomers, C (3), ³J_{PCCC} =11.6, ³J_{PCCC}=11.7), 21.40 and 21.37 (2d from 2 diastereomers, P*C*H₂CH₃, J_{PC}=101.7, J_{PC}=101.4), 16.78 (d, POCH₂*C*H₃, ³J_{POCC}=6.3), 5.85 (d, PCH₂CH₃, ²J_{PCC}=4.8); LREIMS: 204.1 (M).

### (±)-Ethyl(4-hydroxycyclopent-1-enyl)isopropylphosphinate (12)

¹H NMR (CDCl₃): δ 6.64 (dm, 1H, J=9.2), 4.65 (m, 1H,), 4.05 (m, 2H), 2.80 (m, 2H), 2.60 (s, 1H), 2.53 (m, 2H), 1.93 (m, 1H), 1.33 (dt, 3H, J=1.9, J=7.0), 1.21 (d, 3H, J=7.2), 1.16 (d, 3H, J=7.1); ¹³C NMR (CDCl₃): δ 146.08 (t, C (2), ²J_{PCC}=9.4), 132.72 (d, C (1), J_{PC}=119.9), 72.21 (d, C (4), ³J_{PCCC}=9.9), 60.73 and 60.71 (2d from 2 distereomers, PO*C*H₂CH₃, ²J_{POC}=6.8, J=6.8), 44.37 (d, C (5), ²J_{PCC}=15.2), 44.23 and 44.14 (2d from 2 diastereoamers, C (3), ³J_{PCCC}=11.0, J=11.1), 27.46 (d, PO*C*H (CH₃)₂, J_{PC}=102.3), 16.80 (d, POCH₂*C*H₃, ³J_{POCC}=6.3), 15.40 and 15.37 (2d from 2 diastereomers, ²J_{PCC}=25.7, ²J_{PCC}=28.0); LREIMS: 218.1 (M).

### (±)-Ethyl(4-hydroxycyclopent-1-enyl)butylphosphinate (13)

(99%) ¹H NMR (CDCl₃): δ 6.62 (dm, 1H, J=10.1), 4.64 (m, 1H), 4.08 (m, 2H), 2.81 (m, 2H), 2.53 (m, 2H), 1.75 (m, 2H), 1.56 (m, 2H), 1.41 (hept, 2H, J=7.3), 1.32 (dt, 3H, J=2.0, J=7.0), 0.92 (t, 1H, J=7.2); ¹³C NMR (CDCl₃): δ 145.31 and 145.12 (2d from 2 diastereomers, C (2), ²J_{PCC}=10.7, ²J_{PCC}=10.8), 133.76 (d, C (1), J_{PC}=125.6), 72.01 (d, C (4), ³J_{PCCC}=10.5), 60.56 (d, PO*C*H₂CH₃, ²J_{POC}=6.3), 44.38 (d, C (5), ²J_{PCC}=15.6), 43.61 and 43.55 (2d from 2 diastereomers, C (3), ³J_{PCCC}=11.6, ³JP_{CCC}=11.7), 28.14 (d, P*C*H₂ (CH₂)₂CH₃, J_{PC}=100.3), 23.99 (d, PCH₂*C*H₂CH₂CH₃, ²J_{PCC}=16.3), 23.86 (d, P (CH₂)₂*C*H₂CH₃, ³J_{PCCC}=3.7), 16.79 (d, POCH₂*C*H₃, ²J_{POC}=6.3), 13.83 (P (CH₂)₃*C*H₃); LREIMS: 232.1.

### (R)-Ethyl(4-hydroxycyclopent-1-enyl)butylphosphinate

¹H NMR (CDCl₃): δ 6.61 (m, 1H), 4.63 (m, 1H), 3.99 (m, 2H), 2.78 (m, 2H), 2.52 (m, 2H), 1.75 (m, 2H), 1.54 (ddt, 2H, J=6.7, J=9.6, J=16.3), 1.41 (m, 2H), 1.31 (t, 3H, J=7.1), 0.92 (t, 3H, J=7.2); ¹³C NMR (CDCl₃): δ 145.36 and 145.16 (2d from 2 diastereomers, C (2), ²J_{PCC} =11.0, ²J_{PCC} =10.7), 133.73 (d, C (1), J_{PC} =124.3), 72.04 (d, C (4), ³J_{PCCC}=10.4), 60.57 (d, PO*C*H₂CH₃, ²J_{POC}=5.8), 44.38 (d, C (5), ²J_{PCC}=15.6), 43.56 and 43.61 (2d from 2 diastereomers, C (3), ³J_{PCCC}=11.7, ³J_{PCCC} =11.6), 28.13 (d, P*C*H₂ (CH₂)₂CH₃, J_{PC}=100.3), 24.10 (d, PCH₂*C*H₂CH₂CH₃, ²J_{PCC}=16.3), 23.73 (d, P (CH₂)₂*C*H₂CH₃, ³J_{PCCC}=3.6), 16.78 (d, POCH₂*C*H₃, ²J_{POC}=6.4), 13.82 (P (CH₂)₃*C*H₃); LREIMS: 232.1.

### (S)-Ethyl(4-hydroxycyclopent-1-enyl)butylphosphinate

¹H-NMR (CDCl₃): δ 6.61 (m, 1H), 4.63 (m, 1H), 3.99 (m, 2H), 2.78 (m, 2H), 2.52 (m, 2H), 1.75 (m, 2H), 1.54 (ddt, 2H, J=6.7, J=9.6, J=16.3), 1.41 (m, 2H), 1.31 (t, 3H, J=7.1), 0.92 (t, 3H, J=7.2); ¹³C-NMR (CDCl₃): δ 145.36 and 145.16 (2d from 2 diastereomers, C (2), ²J_{PCCC}=11.0, ²J_{PCC} =10.7), 133.73 (d, C (1), J_{PC}=124.3), 72.04 (d, C (4), ³J_{PCCC}=10.4), 60.57 (d, PO*C*H₂CH₃, ²J_{POC}=5.8), 44.38 (d, C (5), ²J_{PCC}=15.6), 43.56 and 43.61 (2d from 2 diastereomers, C (3), ³J_{PCCC}=11.7, ³J_{PCCC} =11.6), 28.13 (d, P*C*H₂ (CH₂)₂CH₃, J_{PC}=100.3), 24.10 (d, PCH₂*C*H₂CH₂CH₃, ²J_{PCC}=16.3), 23.73 (d, P (CH₂)₂*C*H₂CH₃, ³J_{PCCC}=3.6), 16.78 (d, POCH₂*C*H₃, ²J_{POC}=6.4), 13.82 (P (CH₂)₃*C*H₃); LREIMS : 232.1.

### (±)-Ethyl(4-hydroxycyclopent-1-enyl)benzylphosphinate (14)

¹H NMR (CDCl₃): δ 1.29 (t, 3, J=7.1), 2.43 (m, 2), 2.72 (m, 2), 3.95 (m, 2), 4.53 (m, 1), 6.55 (bd, 1, J=18.0), 7.1-7.5 (m, 5).

### (±)-(4-Aminocyclopent-1-enyl)methylphosphinic acid (15) (conversion of alcohol to amine via Mitsunobu/Staudinger reaction)

To a solution of (±)-ethyl(4-hydroxycyclopent-1-enyl)methylphosphinate (10) (150mg, 0.85mmol), DIAD (0.7ml, 1.87mmol, 2.2 equiv) and HN₃ (1.9M in benzene, 1.1ml, 1.7mmol, 2equiv) in anhydrous THF (10ml) at 0°C was added triphenylphosphine (0.89g, 3.4mmol, 4 equiv) in small portions over 1 hour. The reaction mixture was allowed to warm to room temperature and stirred for 12 hours. The reaction mixture was then heated to 50°C for 3 hours at which time water (0.2ml) was added and the heating continued for a further 2 hours. The mixture was the cooled to room temperature and the solvent removed in vacuo. The residue was separated between HCl (1M, 10ml) and DCM (10ml). The organic layer was separated and extracted with aqueous HCl (2 x 10ml). The combined aqueous layers were washed with DCM (2x10ml) and concentrated in vacuo. The crude amino ester hydrochloride salt was hydrolysed by refluxing in aqueous HCl (6M, 15ml) for 30 hours after which time the mixture was cooled to room temperature and the solvent removed in vacuo. The residue was purified by ion exchange chromatography (Dowex 50, H⁺), eluting first with water until the eluant was neutral and colourless followed by pyridine (1M). Fractions containing the product were collected and concentrated in vacuo to give (±)-(4-aminocyclopent-1-enyl)methylphosphinic acid as a yellow solid (80mg, 64%). ¹H NMR (D₂O): δ 7.51 (m, 2H), 6.11 (d, 1H, J=9.8), 3.96 (m, 1H), 2.86 (m, 2H), 2.46 (m, 2H), 1.22 (d, 3H, J=14.1); ¹³C-NMR (D₂O): δ 136.8 (d, C(2), ²J_{PCC}=11.5), 135.56 (d, C (1), J_{PC}=118.0), 51.0 (d, C(4), ³J_{PCCO}=10.3), 38.9 (d, C(3), ²J_{PCC}=15.4), 38.2 (d, C(5), ³J_{PCCC}=13.5), 15.9 (d, PCH₃, J_{PC}=99.05).

### (S)-(4-Aminocyclopent-1-enyl)methylphosphinic acid

¹H NMR (D₂O): δ 7.51 (m, 2H), 6.11 (d, 1H, J=9.8), 3.96 (m, 1H), 2.86 (m, 2H), 2.46 (m, 2H), 1.22 (d, 3H, J=14.1); ¹³C-NMR (D₂O): δ 136.8 (d, C(2), ²J_{PCC}=1 1.5),135.56 (d, C (1), J_{PC}=118.0), 51.0 (d, C(4), ³J_{PCCC}=10.3), 38.9 (d, C(3), ²J_{PCC}=15.4), 38.2 (d, C(5), ³J_{PCCC}=13.5), 15.9 (d, P*C*H₃, J_{PC}=99.05).

### (R)-(4-Aminocyclopent-1-enyl)methylphosphinic acid

¹H NMR (D₂O): δ 7.51 (m, 2H), 6.11 (d, 1H, J=9.8), 3.96 (m, 1H), 2.86 (m, 2H), 2.46 (m, 2H), 1.22 (d, 3H, J=14.1); ¹³C-NMR (D₂O): δ 136.8 (d, C(2), ²J_{PCC}=11.5), 135.56 (d, C (1), J_{PC}=118.0), 51.0 (d, C(4), ³J_{PCCC}=10.3), 38.9 (d, C(3), ²J_{PCC}=15.4), 38.2 (d, C(5), ³J_{PCCC}=13.5), 15.9 (d, PCH₃, J_{PC}=99.05).

A similar procedure was used to prepare compounds (16) to (19). The characteristics of these compounds, as well as the enantiopure phosphinic amino acids corresponding to compound (18), are described below.

### (±)-(4-Aminocyclopent-1-enyl)ethylphosphinic acid (16)

¹H NMR (D₂O): δ 6.17 (bd, 1, J=9.2), 6.17 (bd, 1, J=9.2), 3.95 (bsept, 1), 2.86 (bdd, 2, J=7.4, 16.5), 2.46 (bd, 2, J=18.1), 1.63 (dsept, 1, J=12.15), 1.48 (m, 2), 0.81(t, 3, J=6.9).

### (±)-(4-Aminocyclopent-1-enyl)isopropylphosphinic acid (17)

¹H NMR (D₂O): δ 7.55 (m, 2H), 6.17 (dm, 1H, J=9.1), 3.95 (m, 1H), 2.86 (dd, 2H, J=7.5, J=16.8), 2.47 (d, 2H, J=16.2), 1.63 (m, 1H), 0.94 (t, 3H, J=7.1), 0.88 (t, 3H, J=7.2); ¹³C NMR (D₂O): δ 138.87 (d, C (2), ²J_{PCC}=9.3), 137.56 (d, C (1), J_{PC}=120.0), 50.98 (d, C (4), ³J_{PCCC}=9.4), 38.98 (apparent t, C (3) and C (5), ³J_{PCC}=13.8), 27.84 (d, P*C*H (CH₃)₂, J_{PC}=101.1), 15.19 (dd, PCH (*C*H₃)₂, J=2.3, J=27.0).

### (±)-(4-Aminocyclopent-1-enyl)butylphosphinic acid (18)

¹H NMR (D₂O): δ 7.54 (m, 2H), 6.12 (dm, 1H, J=9.2), 3.95 (m, 1H) 2.85 (dd, 2H, J=7.7, J=16.1), 2.46 (dd, 2H, J=3.4, J=18.2), 1.47 (m, 2H), 1.27 (m, 4H), 0.76 (t, 1H, J=7.1); ¹³C NMR (D₂O): δ 137.68 (d, C (2), ²J_{PCC}=10.2), 132.23 (d, C (1), J_{PC}=121.8), 50.94 (d, C (4), J=10.1), 38.90 (d, C (3), ³J_{PCCC}=14.8), 38.53 (d, C (5), ²J_{PCC}=12.9), 29.61 (d, P*C*H₂ (CH₂)₂CH₃, J=98.7), 24.24 (d, P (CH₂)₂*C*H₂CH₃, ³J_{PCCC}=3.6), 23.74 (d, PCH₂*C*H₂CH₂CH₃, ²J_{PCC}=16.1), 13.15 (s, P (CH₂)₃CH₃).

### (S)-(4-Aminocyclopent-1-enyl)butylphosphinic acid

¹H NMR (D₂O): δ 7.54 (m, 2H), 6.12 (dm, 1H, J=9.2), 3.95 (m, 1H) 2.85 (dd, 2H, J=7.7, J=16.1), 2.46 (dd, 2H, J=3.4, J=18.2), 1.47 (m, 2H), 1.27 (m, 4H), 0.76 (t, 1H, J=7.1); ¹³C NMR (D₂O): δ 137.68 (d, C (2), ²J_{PCC}=10.2),132.23 (d, C (1), J_{PC}=121.8), 50.94 (d, C (4), J=10.1), 38.90 (d, C (3), ³J_{PCCC}=14.8),38.53 (d, C (5), ²J_{PCCC}=12.9), 29.61 (d, P*C*H₂ (CH₂)₂CH₃, J=98.7), 24.24 (d, P (CH₂)₂*C*H₂CH₃, ³J_{PCCC}=3.6), 23.74 (d, PCH₂*C*H₂CH₂CH₃, ²J_{PCC}=16.1), 13.15 (s, P (CH₂)₃CH₃).

### (R)-(4-Aminocyclopent-1-enyl)butylphosphinic acid

¹H NMR (D₂O): δ 7.54 (m, 2H), 6.12 (dm, 1H, J=9.2), 3.95 (m, 1H) 2.85 (dd, 2H, J=7.7, J=16.1), 2.46 (dd, 2H, J=3.4, J=18.2), 1.47 (m, 2H), 1.27 (m, 4H), 0.76 (t, 1H, J=7.1); ¹³C NMR (D₂O): δ 137.68 (d, C (2), ²J_{PCC}=10.2), 132.23 (d, C (1), J_{PC}=121.8), 50.94 (d, C (4), J=10.1), 38.90 (d, C (3), ³J_{PCCC}=14.8), 38.53 (d, C (5), ²J_{PCC}=12.9), 29.61 (d, P*C*H₂ (CH₂)₂CH₃, J=98.7), 24.24 (d, P (CH₂)₂*C*H₂CH₃, ³J_{PCCC}=3.6), 23.74 (d, PCH₂*C*H₂CH₂CH₃, ²J_{PCC}=16.1), 13.15 (s, P (CH₂)₃CH₃).

### (±)-(4-Aminocyclopent-1-enyl)benzylphosphinic acid (19)

¹H NMR (D₂O): δ 7.1-7.6 (m, 6), 6.07 (bd, 1, J=9.2), 3.88 (m, 1), 2.73 (bdd, 2, J=5.0, J=38.2), 2.38 (bt, 2, J=19.0).

### Example 2 - Electrophysiology

The activity of compounds (15), (16), (17), (18) and (19) on GABA receptor sub-types were determined as described in Chebib, M., Mewett, K.N., Johnston, G.A.R., 1998, "GABAC receptor antagonists differentiate between human ρ1 and ρ2 receptors expressed in Xenopus oocytes", European Journal of Pharmacology 357, 227-234.
The results are set out in Table 1.

In this method, oocytes were harvested from *Xenopus laevis* and defolliculated. The oocytes were then stored in ND96 solution (96 mM NaCl, 2 mM KCl, 1.8 mM CaCl₂.2H₂O, 1 mM MgCl₂.6H₂O, 5 mM HEPES (hemi-Na), pH 7.5) supplemented with 2.5 mM sodium pyruvate, 0.5 mM theophylline and 50 µgml⁻¹ gentamycin. The storage solution was changed daily.

Stage V-VI oocytes were injected with mRNA (10 ng / 50 nl) and then stored at 16°C. Recordings of receptor activity were obtained after two to eight days by a two-electrode voltage clamp by means of a Geneclamp 500 amplifier (Axon Instruments Inc., Foster City, CA), a MacLab 2e recorder (AD Instruments, Sydney, NSW) and Chart version 3.6.3 program. Oocytes were voltage clamped at -60 mV and the preparation was continually perfused with ND96 solution at room temperature. Known concentrations of agonists and antagonists dissolved in ND96 were applied in the absence and presence of GABA respectively until maximum current was reached, at which time the oocyte was washed for five to ten minutes to allow complete recovery of response to a known dose of GABA. In the case of antagonists, two dose response curves were conducted on the same cell to enable calculation of single point estimates: a GABA dose-response curve and a GABA dose-response curve in the presence of a known concentration of antagonist. All compounds were tested on oocytes from at least three harvests.

**Table 1: Activity of Compounds on GABA receptor subtypes**

| Compound | GABA_{A} (α1β2γ2L) | GABA_{B} (GB1) | GABA_{C} (ρ1) | Selectivity |
|---|---|---|---|---|
| 15 | Antagonist | Partial Agonist | Antagonist | GABA_{C} |
| | (IC₅₀ > 300µM) | (EC₅₀ = 44.62 µM ± 0.23µM) | (IC₅₀ = 0.85µM ± 0.23µM) | ~50x |
| 16 | Antagonist | Antagonist | Antagonist | GABA_{C} |
| | (IC₅₀ > 600µM) | (IC₅₀ > 300µM) | (IC₅₀ = 12.32 µM ± 3.97µM) | ~25x |
| 17 | Antagonist | Antagonist | Antagonist | GABA_{C} |
| | (IC₅₀ > 600µM) | (IC₅₀ > 300µM) | (IC₅₀ = 6.34 ± 2.12µM) | ~50x |
| 18 | Antagonist | Antagonist | Antagonist | GABA_{C} |
| | (IC₅₀ > 300µM) | (IC₅₀ ~ 300µM) | (IC₅₀ = 3.68µM± 0.67 µM) | ~100x |
| 19 | Antagonist | (No effect - 300µM) | Antagonist | |
| | | | (IC₅₀ > 300µM) | |

The results set out in Table 1 demonstrate that the compounds of the present invention are highly selective GABA_{c} receptor antagonists. The compounds of the present invention are much more selective GABAc receptor antagonists than compound (19).

### Example 3 - Comparison with (±)-(3-aminocvclopent-1-enyl)methylphosphinic acid

(±)-(3-Aminocyclopent-1-enyl)methylphosphinic acid was prepared as described below. This compound is similar to compound (15) referred to above, but the amino group is at the 3-position of the pentene ring rather than the 4-position.

(±)-Ethyl(3-aminocyclopentenyl)methylphosphinate was prepared as described in WO 03/045897 and was dissolved in aqueous HCl (6M, 40 ml) and the solution heated to reflux for 30 h. After cooling, the solution was evaporated to dryness under reduced pressure. The residue was dissolved in water (10 ml) and applied to an ion exchange column (Dowex 50, H⁺ form). The column was washed with water until the washings were colourless and pH neutral and then eluted with aqueous pyridine (1M). Ninhydrin positive fractions were combined and evaporated to dryness under reduced pressure. Residual traces of pyridine were removed by repeatedly redissolving the compound in water (20 ml) and re-evaporating (3 times). Recrystallisation from ethanol/acetone and drying gave (±)-(3-aminocyclopent-1-enyl)methylphosphinic acid.

The activity of this compound on the GABA receptor sub-types was determined by the same procedure as described in Example 2 for compounds (**15**) to (**19**). (±)-3-Aminocyclopente-1-nyl)methylphosphinic acid (200 µM) had no effect alone on ρ1 GABA_{C} receptors but in the presence of 1 µM GABA inhibited the response by 65%. The compound is therefore 50-100 times less active at GABA_{C} than compound (**15**).

As can be seen from these results, compound (**15**) of the present invention is a much more active GABA_{C} receptor antagonist than (±)-(3-aminocyclopent-1-enyl)methyl phosphinic acid. This result demonstrates that the position of the double bond in the cyclopentene ring greatly effects the selectivity of the compounds of the present invention.

The compounds of the present invention are selective GABA_{C} receptor antagonists, and can therefore be used to enhance the cognitive activity of animals, including humans. The ability of the compounds of the present invention to enhance cognitive activity in an animal can be demonstrated by tests known in the art, such as the Morris Water Maze test and the day-old chick model of memory (Gibbs & Ng 1977; Gibbs & Summers, 2002). Such tests can also be used to determine effective dosages of a selective GABA_{C} receptor antagonist for enhancing the cognitive activity of an animal, and as an animal model for determining suitable effective dosages for humans.

It will be apparent to the person skilled in the art that while the invention has been described in some detail for the purposes of clarity and understanding, various modifications and alterations to the embodiments described herein may be made without departing from the scope of the inventive concept disclosed in the specification.

In the claims which follow and in the preceding description of the invention, except where the context requires otherwise due to express language or necessary implication, the word "comprising" is used in an inclusive sense, i.e. to specify the presence of the stated features but not to preclude the presence or addition of further features in various embodiments of the invention.
Chebib M., Vandenberg RJ., Froestl W. and Johnston G.A.R. Eur. J. Pharmacol., 1997 329, 223-229
Chebib M. Chemistry in Australia, October 2001, 19-21
Chebib M. and Johnston G.A.R. J. Med. Chem., 2000 43, 1427-1447
Drew C. A., Johnston G. A. R. and Weatherby R. P. Neurosci. Let. 1984 52, 317-321
Feigenspan A. Wössle H. and Bormann J. Nature, 1993 361, 159-162
Froestl W., Mickel, S. J., von Sprecher, G., Diel. P. J., Hall, R. G., Maier, L., Strub, D., Melillo, V., Baumann, P. A., Bernasconi, R, Gentsch, C., Hauser, K., Jaekel, J., Karlsson, G., Klebs, K., Maitre, L., Marescaux, C., Pozza, M. F., Schmutz, M., Steinmann, M. W., Riezen, H., Vassout, A., Mondadori, C., Olpe, H-R., Waldmeier, P.C., and Bittiger, H. J. Med. Chem., 1995, 38, 3313-3331
Gibbs, M. E. and Ng, K. T., 1977. Psychobiology of memory: towards a model of memory formation. Biobehav. Reviews, 1,113-136.
Gibbs, M. E. and Summers, R J., 2002. Role of adrenoceptor subtypes in memory consolidation. Prog Neurobiol., 67, 345-391
Irwin, S. "Comprehensive Observational Assessment: la. A Systematic, Quantitative Procedure for Assessing the Behavioural and Physiologic State of the Mouse", Psychopharmacologia, 1968 13, 222-257
Johnston, G.A.R., Curtis, D.R., Beart, P.M., Game, C.J.A., McCulloch, R.M. and Twitchin, B. J. Neurochem., 1975 24, 157-160
Johnston, G.A.R., Allan, R.D., Kennedy, S.M.E. and Twitchin, B. "GABA-Neurotransmitters", Alfred Benzon Symposium 12, Munksgaard, 1978, p 149-164
Johnston, G.A.R Phamacol. Ther., 1996 69, 173-198 ("Johnston, 1996a")
Johnston, G.A.R. Trends Pharmacol. Sci., 1996 17, 319-323 ("Johnston, 1996b")
Kerr, D.I.B. and Ong, J. Pharmacol. Ther., 1995 67, 187-246
Murata, Y., Woodward, R.M., Miledi, R. and Overman, L.E. Bioorg. and Med Chem. Lett., 1996 6, 2073-2076
Piers, E., Grierson, J.R, Lau, C.K. and Nagakura, I. Can. J. Chem. 1982, 60, 210
Qian, H. and Dowling, J.E. Nature, 1993 361, 162-164
Ragozzino, D., Woodward, R.M., Murata, F., Eusebi, F., Overman, L.E. and Miledi, R. Mol. Pharmacol., 1996 50, 1024-1030
Woodward, R.M., Polenzani, L. and Miledi, R Mol. Pharmacol., 1993 43, 609-625

## Claims

1. A compound of the formula I: 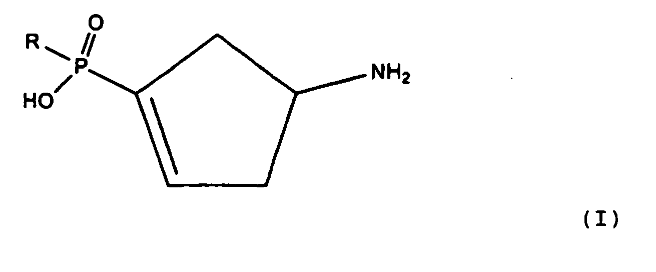 wherein R is methyl, ethyl, propyl, isopropyl, butyl, pentyl, neo-pentyl or cyclohexyl, or a salt or solvate thereof.

2. An *in vitro* method of selectively antagonising GABAc receptors compared to GABA_{B} or GABA_{A} receptors, comprising the step of exposing the receptors to an effective amount of a compound of formula I as defined in claim 1 or a pharmaceutically acceptable salt or solvate thereof.

3. Use of a compound of formula I as defined in claim 1 or a pharmaceutically acceptable salt or solvate thereof for the manufacture of a medicament for selectively antagonising GABA_{C} receptors compared with GABA_{B} or GABA_{A} receptors.

4. Use of a compound of formula I as defined in claim 1 or a pharmaceutically acceptable salt or solvate thereof for the manufacture of a medicament for enhancing the cognitive activity of an animal.

5. Use of a compound of formula I as defined in claim 1 or a pharmaceutically acceptable salt or solvate thereof for the manufacture of a medicament for stimulating memory capacity in an animal.

6. Use according to any one of claim 3-5, wherein the animal is suffering from one or more conditions selected from the group consisting of cognitive deficit, memory impairment and dementia.

7. Use according to any one of claims 3-6, wherein the animal is suffering from dementia, Alzheimer's disease, AIDS or schizophrenia.

8. Use according to any one of claims 3-7, wherein the animal is a human.

9. A pharmaceutical composition comprising a compound of formula I as defined in claim 1 or a pharmaceutically acceptable salt or solvate thereof, and a pharmaceutically acceptable carrier.

## Patentansprüche

1. Verbindung der Formel I: 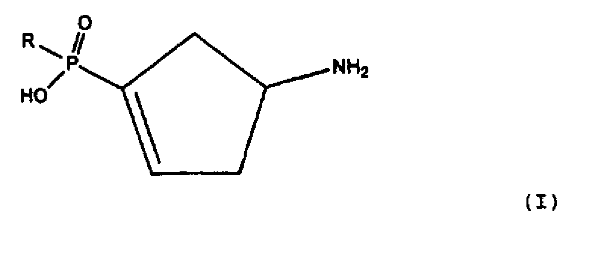 wobei R Methyl, Ethyl, Propyl, Isopropyl, Butyl, Pentyl, neo-Pentyl oder Cyclohexyl ist, oder ein Salz oder Solvat davon.

2. Ein in vitro Verfahren zur selektiven Antagonisierung von GABA_{C}-Rezeptoren im Vergleich zu GABA_{B}- oder GABA_{A}-Rezeptoren, umfassend den Schritt der Exposition der Rezeptoren gegenüber einer wirksamen Menge einer Verbindung der Formel I, wie in Anspruch 1 definiert, oder ein pharmazeutisch verträgliches Salz oder Solvat davon.

3. Verwendung einer Verbindung der Formel I, wie in Anspruch 1 definiert, oder ein pharmazeutisch verträgliches Salz oder Solvat davon zur Herstellung eines Medikaments zur selektiven Antagonisierung von GABA_{C}-Rezeptoren im Vergleich mit GABA_{B}- oder GABA_{A}-Rezeptoren.

4. Verwendung einer Verbindung der Formel I, wie in Anspruch 1 definiert, oder eines pharmazeutisch verträglichen Salzes oder Solvates davon zur Herstellung eines Medikaments zur Verstärkung der kognitiven Aktivität eines Tiers.

5. Verwendung einer Verbindung der Formel I, wie in Anspruch 1 definiert, oder eines pharmazeutisch verträglichen Salzes oder Solvates davon zur Herstellung eines Medikaments zur Stimulierung der Gedächtniskapazität in einem Tier.

6. Verwendung nach einem der Ansprüche 3 bis 5, wobei das Tier an einem oder mehreren Zuständen leidet, die aus der Gruppe ausgewählt sind, bestehend aus einem kognitiven Mangel, einer Gedächtnisstörung und Demenz.

7. Verwendung nach einem der Ansprüche 3 bis 6, wobei das Tier an Demenz, Alzheimer-Krankheit, AIDS oder Schizophrenie leidet.

8. Verwendung nach einem der Ansprüche 3 bis 7, wobei das Tier ein Mensch ist.

9. Pharmazeutische Zusammensetzung, die eine Verbindung der Formel I, wie in Anspruch 1 definiert, oder ein pharmazeutisch verträgliches Salz oder Solvat davon und einen pharmazeutisch verträglichen Träger umfasst.

## Revendications

1. Composé de formule I : 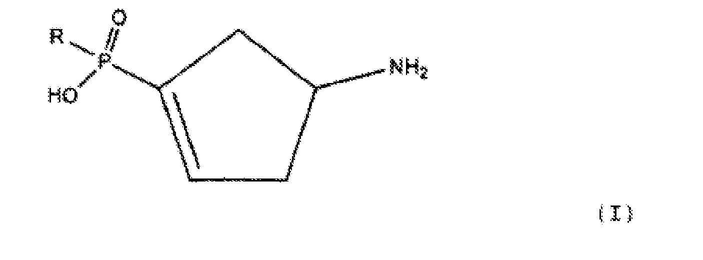 dans laquelle R est un méthyle, un éthyle, un propyle, un isopropyle, un butyle, un pentyle, un néo-pentyle ou un cyclohexyle, ou un sel ou un solvate de celui-ci.

2. Procédé *in vitro* permettant d'obtenir sélectivement un effet antagoniste contre les récepteurs GABA_{C} par rapport aux récepteurs GABA_{B} ou GABA_{A}, comprenant l'étape d'exposition des récepteurs à une quantité efficace d'un composé de formule I selon la revendication 1 ou d'un sel ou d'un solvate de celui-ci pharmaceutiquement acceptable.

3. Utilisation d'un composé de formule I selon la revendication 1 ou d'un sel ou d'un solvate de celui-ci pharmaceutiquement acceptable pour la fabrication d'un médicament permettant d'obtenir sélectivement un effet antagoniste contre les récepteurs GABA_{C} par rapport aux récepteurs GABA_{B} ou GABA_{A}.

4. Utilisation d'un composé de formule I selon la revendication 1 ou d'un sel ou d'un solvate de celui-ci pharmaceutiquement acceptable pour la fabrication d'un médicament destiné à améliorer l'activité cognitive d'un animal.

5. Utilisation d'un composé de formule I selon la revendication 1 ou d'un sel ou d'un solvate de celui-ci pharmaceutiquement acceptable pour la fabrication d'un médicament destiné à stimuler la capacité mémorielle d'un animal.

6. Utilisation selon l'une quelconque des revendications 3 à 5, dans laquelle l'animal souffre d'un ou de plusieurs états pathologiques choisis dans le groupe constitué par le déficit cognitif, l'affaiblissement de la mémoire et la démence.

7. Utilisation selon l'une quelconque des revendications 3 à 6, dans laquelle l'animal souffre de démence, de la maladie d'Alzheimer, du SIDA ou de schizophrénie.

8. Utilisation selon l'une quelconque des revendications 3 à 7, dans laquelle l'animal est un humain.

9. Composition pharmaceutique comprenant un composé de formule I selon la revendication 1 ou un sel ou un solvate de celui-ci pharmaceutiquement acceptable, et un support pharmaceutiquement acceptable.
